# EUROPÄISCHE PATENTSCHRIFT

(11) **EP 1 957 658 B1**
(45) Veröffentlichungstag und Bekanntmachung des Hinweises auf die Patenterteilung: **20.07.2011**
(21) Anmeldenummer: 06819766.4
(22) Anmeldetag: 27.11.2006
(51) Int. Cl.: C12P 19/14, C12P 13/08, C12P 13/14

(54) **FERMENTATIVE HERSTELLUNG ORGANISCHER VERBINDUNGEN UNTER EINSATZ DEXTRIN-HALTIGER MEDIEN**
FERMENTATIVE PRODUCTION OF ORGANIC COMPOUNDS USING SUBSTANCES CONTAINING DEXTRIN
PREPARATION PAR FERMENTATION DE COMPOSES ORGANIQUES PAR UTILISATION DE SUBSTANCES CONTENANT DE LA DEXTRINE

(30) Priorität: 28.11.2005 DE 102005056669
(43) Veröffentlichungstag der Anmeldung: 20.08.2008
(73) Patentinhaber: BASF SE, 67056 Ludwigshafen (DE)
(72) Erfinder: BOY, Matthias, 63225 Langen (DE); FREYER, Stephan, 67434 Neustadt (DE)
(86) Internationale Anmeldenummer: PCT/EP2006/068927
(87) Internationale Veröffentlichungsnummer: WO 2007/060234

(56) Entgegenhaltungen:
- EP-A2- 0 171 218
- WO-A-2004/113551
- JP-A- 2001 309 751

## Beschreibung

Die vorliegende Erfindung betrifft die fermentative Herstellung organischer Verbindungen mit mindestens 3 C-Atomen oder mit mindestens 2 C-Atomen und mindestens 1 N-Atom unter Einsatz eines Dextrin-haltigen Mediums, das zumindest einen Teil der nicht-stärkehaltigen festen Bestandteile der Stärkequelle umfasst, zur Kultivierung der Mikroorganismen.

Zuckerhaltige Flüssigmedien sind eine grundlegende Nährstoffquelle für viele Fermentationsverfahren; die in den Medien enthaltenen Zuckeranteile werden von den eingesetzten Mikroorganismen verstoffwechselt, wobei man organische Wertprodukte erhält. Die Palette derart hergestellter mikrobieller Stoffwechselprodukte, d. h. organischer Verbindungen, umfasst hierbei z. B. niedermolekulare flüchtige Verbindungen wie Ethanol, nichtflüchtige Stoffwechselprodukte wie Aminosäuren, Vitamine und Carotenoide sowie eine Vielzahl weiterer Stoffe.

Für solche allgemein bekannten mikrobiellen Fermentationsverfahren werden in Abhängigkeit von den verschiedenen Verfahrensbedingungen unterschiedliche Kohlenstoffquellen genutzt. Diese reichen von reiner Saccharose über Rüben- und Zuckerrohrmelasse, so genannten "high test molasses" (invertierte Zuckerrohrmelasse) bis hin zu Glucose aus Stärkehydrolysaten. Für die biotechnologische Herstellung von L-Lysin werden darüber hinaus Essigsäure und Ethanol als großtechnisch einsetzbare Co-Substrate genannt (Pfefferle et al., Biotechnogical Manufacture of Lysine, Advances in Biochemical Engineering/Biotechnology, Vol. 79 (2003), 59-112).

Auf Basis der genannten Kohlenstoffquellen sind verschiedene Methoden und Vorgehensweisen zur zuckerbasierten, fermentativen Herstellung mikrobieller Stoffwechselprodukte etabliert. Am Beispiel des L-Lysins werden diese beispielsweise von Pfefferle et al. (a.a.O.) im Hinblick auf Stammentwicklung, Prozessentwicklung und großtechnische Produktion beschrieben.

Eine wichtige Kohlenstoffquelle für die durch Mikroorganismen vermittelte fermentative Herstellung mikrobieller Stoffwechselprodukte ist Stärke. Diese muss in vorgelagerten Reaktionsschritten zunächst verflüssigt und verzuckert werden, bevor sie als Kohlenstoffquelle in einer Fermentation genutzt werden kann. Hierzu wird die Stärke aus einer natürlichen Stärkequelle wie Kartoffeln, Cassava, Getreide, z. B. Weizen, Mais, Gerste, Roggen, Triticale oder Reis üblicherweise in vorgereinigter Form gewonnen und anschließend enzymatisch verflüssigt und verzuckert, um dann in der eigentlichen Fermentation zur Produktion der gewünschten Stoffwechselprodukte eingesetzt zu werden.

Neben dem Einsatz derartig vorgereinigter Stärkequellen ist auch die Verwendung nicht aufgereinigter Stärkequellen zur Herstellung von Kohlenstoffquellen für die fermentative Produktion mikrobieller Stoffwechselprodukte beschrieben worden. Typischerweise werden die Stärkequellen dabei zunächst durch Mahlen zerkleinert. Das Mahlgut wird dann einer Verflüssigung und Verzuckerung unterworfen. Da dieses Mahlgut naturgemäß neben Stärke noch eine Reihe von nicht-stärkehaltigen Bestandteilen enthält, welche die Fermentation nachteilig beeinflussen können, werden diese Bestandteile üblicherweise vor der Fermentation abgetrennt. Die Entfernung kann entweder direkt nach dem Mahlen (WO 02/077252; JP 2001-072701; JP 56-169594; CN 1218111), nach der Verflüssigung (WO 02/077252; CN 1173541) oder im Anschluss an die Verzuckerung (CN 1266102; Beukema et al.: Production of fermentation syrups by enzymatic hydrolysis of potatoes; potatoe saccharification to give culture medium (Conference Abstract), Symp. Biotechnol. Res. Neth. (1983), 6; NL8302229) erfolgen. In allen Varianten wird jedoch ein weitgehend reines Stärkehydrolysat in der Fermentation eingesetzt.

Neuere Verfahren zur fermentativen Herstellung von organischen Verbindungen umfassen insbesondere eine Aufreinigung der Stärkequellen vor der Fermentation z. B. die Aufreinigung von verflüssigten und verzuckerten Stärkelösungen (JP 57159500), oder stellen Methoden bereit, welche die Herstellung von Fermentationsmedien aus erneuerbaren Ressourcen (EP 1205557) ermöglichen sollen.

Nicht aufgereinigte Stärkequellen hingegen werden in großem Umfang bei der ferrnentativen Herstellung von Bioethanol eingesetzt. Hierbei werden die Stärkequellen, üblicherweise ganze Getreidekörner, zunächst trocken vermahlen und anschließend der Stärkebestandteil der Stärkequelle unter Einwirkungen von Enzymen hydrolysiert. Dabei kann die Hydrolyse sowohl diskontinuierlich, z. B. in Rührkesseln, als auch kontinuierlich, z. B. In Jet-Kochern durchgeführt werden. Entsprechende Prozessbeschreibungen finden sich z. B. in "The Alcohol Textbook - A reference for the beverage, fuel and industrial alcohol industries", Jaques et al. (Hg.), Nottingham Univ. Press 1995, ISBN 1-8977676-735, Kapitel 2, S. 7 bis 23, und in McAloon et al., "Determining the cost of producing ethanol from corn starch and lignocellulosic feedstocks", NREL/TP-580-28893, National Renewable Energy Laboratory, October 2000.

Da bei der fermentativen Herstellung von Bioethanol das Wertprodukt durch Destillation gewonnen wird, stellt der Einsatz von Stärkequellen aus dem Dry-Milling-Prozess in nicht vorgereinigter Form kein gravierendes Problem dar. Bei der Anwendung eines Dry-Milling-Verfahrens zur Herstellung anderer mikrobieller Stöffwechselprodukte ist jedoch der über die Zuckerlösung in die Fermentation eingetragene Feststoffstrom problematisch, da dieser sich sowohl negativ auf die Fermentation auswirken kann, z. B. im Hinblick auf die Sauerstofftransferrate bzw. den Sauerstoffbedarf der eingesetzten Mikroorganismen (vgl. hierzu Mersmann, A. et al.: Selection and Design of Aerobic Bioreactors, Chem. Eng. Technol. 13 (1990), 357-3701 als auch die anschließende Aufarbeitung nicht unerheblich erschweren kann.

Zudem kann durch den Feststoffeintrag bereits bei der Herstellung der stärkehaltigen Suspension die Viskosität der Suspension einen kritischen Wert erreichen, wodurch z. B. eine Suspension mit mehr als 30 Gew.-% Maismehl in Wasser nicht mehr homogen mischbar ist (Industrial Enzymology, 2. Aufl., T. Godfrey, S. West, 1996). Dadurch ist bei herkömmlichem Vorgehen die Glucosekonzentration begrenzt. Dies ist im Hinblick auf die fermentative Bioethanol-Herstellung insofern nicht weiter relevant, da höhere Konzentrationen aufgrund der Toxizität des Produkts für die zur Fermentation eingesetzten Hefen ohnehin nicht sinnvoll umgesetzt werden könnten.

Bei der fermentativen Herstellung anderer organischer Stoffwechselprodukte als Ethanol ist es prinzipiell von Nachteil, der Fermentation zuckerhaltige Medien mit geringer Zuckerkonzentrationen zuzuführen, weil dies zu einer überproportionalen Verdünnung der Fermentationsbrühe führt und sich folglich die erreichbare Endkonzentration der Wertprodukte verringert, was einerseits erhöhte Kosten bei deren Gewinnung aus dem Fermentationsmedium verursacht und die Raum-Zeit-Ausbeute abnimmt. Diese Erwägungen sind insbesondere für den Fall maßgeblich, wenn ein für eine großvolumige Bioethanol-Produktion hergestelltes Stärkehydrolysat, das herkömmlicherweise geringe Zucker- bzw. Glucosekonzentrationen von bis zu etwa 30 oder 33 Gew.-% aufweist, teilweise einer geringervolumigen Nebenfermentation zur Herstellung anderer Chemikalien zugeführt werden soll.

Andererseits können höhere Konzentrationen an verstoffwechselbaren Monosacchariden im Fermentationsmedium zu einer Inhibierung der Fermentation oder des Wachstums des Mikroorganismus führen oder eine Stoffwechseländerung der eingesetzten Mikroorganismen zur Folge haben. Bei *E.coli* führt beispielsweise eine zu hohe Konzentration freier Glucose z. B. zur Bildung von organischen Säuren (Acetat), während *Saccharomyces cerevisae* in diesem Fall z. B. auf Vergärung umschaltet, obwohl in belüfteten Fermentern ausreichend Sauerstoff vorhanden ist (Crabtree-Effekt). Daher können höhere Konzentrationen an verstoffwechselbaren Monosacchariden in den der Fermation zugeführten Zucker-haltigen Medien während der Zufütterungsphase die Fermentation nachteilig beeinflussen. Problematisch ist auch der Einsatz höher konzentrierter Medien in der Batch-Phase, d. h. während der Wachstumsphase der Mikroorganismen im Fermentationsansatz, bevor der Fermentation über den Feed-Strom weitere Zucker zugeführt werden, da viele Stämme zum Wachsen Glucosekonzentrationen unterhalb 6 Gew.-% benötigen.

Aufgrund der dargestellten Schwierigkeiten und Einschränkungen sind Dry-Milling-Verfahren, wie sie für die Bioethanolherstellung in breitem Umfang eingesetzt werden, bei der fermentativen Herstellung anderer mikrobieller Stoffwechselprodukte als Ethanol bislang ohne wesentliche wirtschaftliche Bedeutung geblieben.

Versuche zur Übertragung des Dry-Milling-Konzepts und der mit diesem Verfahren verbundenen prinzipiellen Vorteile auf die großtechnische Herstellung mikrobieller Stoffwechselprodukte sind bisher lediglich unter Verwendung von Cassava als Stärkequelle beschrieben worden. So beschreibt die JP 2001/275693 ein Verfahren zur fermentativen Herstellung von Aminosäuren, bei dem man als Stärkequelle geschälte Cassavaknollen einsetzt, die trocken vermahlen wurden. Zur Durchführung des Verfahrens ist es jedoch erforderlich, eine Teilchengröße des Mahlgutes von ≤ 150 µm einzustellen. Bei der dazu angewendeten Filtration werden ein Teil des eingesetzten Mahlguts, einschließlich nicht-stärkehaltiger Bestandteile, vor der VerflüssigungNerzuckerung der enthaltenen Stärke und der anschließenden Fermentation abgetrennt. Bei diesem Verfahren werden mäßige Zuckerkonzentrationen erreicht. Ein ähnliches Verfahren wird in der JP 2001/309751 zur Herstellung eines aminosäurehaitigen Futterzusatzes beschrieben.

Erhöhte Zuckerkonzentrationen in dem zur Fermentation eingesetzten Flüssigmedium können bei Einsatz eines Mahlguts zur Verzuckerung, das weitgehend die festen, nicht-stärkehaltigen Bestandteile der Stärkequelle enthält, durch das in der WO 2005/116228 (PCT/EP2005/005728) der Anmelderin beschriebene Verfahren erreicht werden. Eine Abtrennung der in der Stärkequelle enthaltenen festen, nicht-stärkehaltigen Bestandteile vor der Fermentation hat sich dabei überraschenderweise als nicht erforderlich erwiesen. Das beschriebene Verfahren kann unter in-situ-Verzuckerung der verflüssigten Stärkequelle durchgeführt werden. Ein ähnliches Verfahren unter Einsatz von unter Getreidekörnern ausgewählten Stärkequellen wird in der PCT/EP2006/066057 (die ältere Patentanmeldung DE 10 2005 042 541.0) der Anmelderin beschrieben.

Es war daher Aufgabe der vorliegenden Erfindung, ein weiteres Verfahren zur Herstellung organischer Verbindungen durch Fermentation bereitzustellen, das keine oder zumindest keine vollständige vorherige Abtrennung der in der Stärkequelle enthaltenen, nicht-stärkehaltigen festen Bestandteile erfordert. Das Verfahren sollte insbesondere einen vergleichsweise geringen apparativen Aufwand erfordern und den Einsatz von Medien mit hoher Zuckerkonzentrationen ermöglichen. Außerdem sollte es sich durch eine leichte Handhabbarkeit der verwendeten Medien und deren problemlosen Einsatz in der Fermentation auszeichnen. Insbesondere sollte das Verfahren die Verwendung von Getreide als Stärkequelle erlauben.

Es wurde überraschend gefunden, dass ein fermentatives Verfahren zur Herstellung organischer Verbindungen trotz des inhärent hohen Feststoffeintrags effizient durchführbar ist, indem man durch Vermahlen und Verflüssigen von Stärkequellen ohne vorheriges vollständiges Abtrennen der nicht-stärkehaltigen festen Bestandteile der Stärkequelle ein Dextrin-haltiges Medium (1) herstellt und dieses ohne Zugabe verzuckernder Enzyme in einer Fermentation einsetzt.

Gegenstand der Erfindung ist somit ein Verfahren zur Herstellung wenigstens einer organischen Verbindung mit mindestens 3 C-Atomen oder mit mindestens 2 C-Atomen und mindestens 1 N-Atom durch Fermentation, umfassend die folgenden Schritte:
a1) Vermahlen einer Stärkequelle, wobei man ein Mahlgut erhält, das wenigstens einen Teil der nicht-stärkehaltigen festen Bestandteile der Stärkequelle enthält;
a2) Suspendieren des Mahlguts mit einer wässrigen Flüssigkeit und Verflüssigen des in der wässrigen Flüssigkeit enthaltenen Mahlguts in Gegenwart mindestens eines Stärke verflüssigenden Enzyms, wobei man ein wässriges Dextrin-haltiges Medium (1) erhält, das wenigstens einen Teil der nicht-stärkehaltigen festen Bestandteile der Stärkequelle umfasst; und
b) Verwendung des wässrigen Dextrin-haltigen Mediums (1) in einer Fermentation zur Kultivierung eines Mikroorganismus, welcher zur Überproduktion der organischen Verbindung befähigt ist und der ausgewählt ist unter Mikroorganismen, die Enzyme produzieren, welche Dextrine zu Monosacchariden hydrolysieren;
wobei Enzyme, die Dextrine zu Monosacchariden hydrolysieren, nicht oder in einer Menge von weniger als 0,001 Gew.-%, bezogen auf das Gesamtgewicht der eingesetzten Stärkequelle, zugegeben werden.

Das erfindungsgemäße Fermentationsverfahren kann trotz des Gehalts an festen, nicht-stärkehaltigen Bestandteilen der eingesetzten Stärkequelle in dem Dextrin-haltigen Medium (1) effizient durchgeführt werden, ohne dass es der Zugabe verzuckernder Enzyme bedarf. Geringe Mengen, die zu einer vollständigen Verzuckerung nicht ausreichen, typischerweise weniger als 0,001 Gew.-%, insbesondere weniger als 0,0005 Gew.-%, bezogen auf das Gesamtgewicht der eingesetzten Stärkequelle, können jedoch zugegeben werden.

Aufgrund der Verwendung von Dextrinen zur Kultivierung des Mikroorganismus kann eine hohe Konzentration an verstoffwechselbaren Zuckern im Fermentationsmedium sowohl in der Batch-Phase als auch in der Zufütterungsphase eingestellt werden, ohne dass es zu unerwünschten Nebenreaktionen kommt, so dass eine unerwünschte Verdünnung der Fermentationsbrühe vermieden wird. Zudem werden durch das erfindungsgemäße Verfahren Viskositätsprobleme, wie sie beim Verflüssigen der Stärkequelle bei höheren Konzentrationen an Mahlgut auftreten können, weitgehend vermieden.

Hier und im Folgenden werden die Ausdrücke "Dextrin-haltiges Medium" und "Dextrin-haltige Flüssigkeit" synonym verwendet. Es versteht sich für den Fachmann, dass der in der Fermentation eingesetzte Mikroorganismus befähigt sein muss, die in dem wässrigen Dextrin-haltigen Medium (1) enthaltenen Dextrine zu verstoffwechseln, ohne dass diese durch externen Zusatz verzuckernder Enzyme in Di- und/oder Monosacchariden hydrolysiert werden müssen. Dabei werden die Dextrine von dem Mikroorganismus verstoffwechselt, vermutlich nachdem sie durch Stamm-eigene verzuckernde Enzyme, z. B. Stamm-eigene Glucoamylasen, hydrolysiert wurden. Besonders vorteilhaft an dem erfindungsgemäßen Verfahren ist im letzteren Fall, dass während der Fermentation die Geschwindigkeit der Verzuckerung, insbesondere einer Glucosefreisetzung, einerseits durch die Menge an Biomasse und anderseits durch das Expressionsniveau der Stamm-eigenen verzuckernden Enzyme dem Bedarf der Mikroorganismen automatisch angepasst wird.

Der Begriff "Verflüssigung" bedeutet hier und im Folgenden den hydrolytischen Abbau von Stärke zu Oligosacchariden, insbesondere zu Dextrinen.

Die Begriffe "Verzuckerung" bzw. "Verzuckern" bedeuten hier und im Folgenden die Hydrolyse von Dextrinen zu Monosacchariden, insbesondere zu Monosacchariden wie Glucose. Unter einem "verzuckernden Enzym" wird folglich ein Enzym verstanden, das Dextrine zu Monosacchariden hydrolysiert.

Unter dem Begriff "Dextrin" werden hier und im Folgenden durch hydrolytischen Abbau von Stärke erhaltene Oligosaccharide verstanden, die in der Regel aus 3 bis 18, insbesondere 6 bis 12 Monosaccharid-Einheiten, insbesondere aus Glucose-Einheiten, bestehen.

Die Begriffe "Gehalt an Glucoseäquivalenten" und "Zuckerkonzentration" bezeichnet die Gesamtkonzentration an Mono-, Di- und Oligosacchariden im Medium, die potenziell für eine Fermentation zur Verfügung steht. Der Begriff "Glucoseäquivalente" umfasst auch die von Glucose verschiedenen verstoffwechselbaren Zucker bzw. Zuckereinheiten.

Die Begriffe "überproduzierend" bzw. "Überproduktion" werden hier und im Folgenden in Bezug auf einen Mikroorganismus verwendet, um dessen Eigenschaft zu bezeichnen, ein oder mehrere seiner Stoffwechselprodukte in einer Menge zu produzieren, die über die zur Vermehrung des Mikroorganismus benötigte Menge hinausgeht, wodurch es zur Anreicherung im Fermentationsmedium kommt, wobei die Anreicherung extra- oder intrazellulär erfolgen kann. _

Als Stärkequelle kommen für das erfindungsgemäße Verfahren vor allem trockene Kornfrüchte oder Samen in Betracht, die in getrocknetem Zustand mindestens 40 Gew.-% und bevorzugt mindestens 50 Gew.-% Stärkeanteil aufweisen. Diese finden sich in vielen der heutzutage in großem Maßstab kultivierten Getreidepflanzen wie Mais, Weizen, Hafer, Gerste, Roggen, Triticale, Reis sowie in Zuckerrüben, Kartoffeln, Cassava und verschiedenen Hirsesorten, z. B. Sorghum und Milo. Bevorzugt ist die Stärkequelle unter Getreide und speziell unter Mais-, Roggen-, Triticale- und Weizenkörnern ausgewählt. Grundsätzlich lässt sich das erfindungsgemäße Verfahren auch mit analogen Stärkequellen durchführen, wie beispielsweise einer Mischung verschiedener stärkehaltiger Kornfrüchte oder Samen.

Zur Herstellung der Dextrin-haltigen Flüssigkeit wird im Schritt a1) die jeweilige Stärkequelle mit oder ohne Zusatz von Flüssigkeit, z. B. Wasser, vermahlen, bevorzugt ohne Zusatz von Flüssigkeit. Es kann auch eine Trockenvermahlung mit einer anschließenden Nassvermahlung kombiniert werden.

Zur trockenen Vermahlung werden typischerweise Hammermühlen, Rotormühlen oder Walzen-Schrotmühlen eingesetzt; zur Nassvermahlung eignen sich Rührmixer, Rührwerkskugelmühlen, Zirkulationsmühlen, Scheibenmühlen, Ringkammermühlen, Schwingmühlen oder Planetenmühlen. Grundsätzlich kommen auch andere Mühlen in Betracht. Die zur Nassvermahlung erforderliche Flüssigkeitsmenge kann der Fachmann in Routineexperimenten ermitteln. Üblicherweise wird sie so eingestellt, dass der Gehalt an Trockensubstanz im Bereich von 10 bis 20 Gew.-% liegt.

Durch das Mahlen wird eine für die sich anschließenden Verfahrensschritte geeignete Korngröße eingestellt. Hierbei hat es sich als vorteilhaft erwiesen, wenn das beim Vermahlen, insbesondere bei trockener Vermahlung, im Schritt a1) erhaltene Mahlgut Mehlpartikel, d. h. teilchenförmige Bestandteile, mit einer Korngröße im Bereich von 100 bis 630 µm in einem Anteil von 30 bis 100 Gew.-%, bevorzugt 40 bis 95 Gew.-%. und besonders bevorzugt 50 bis 90 Gew.-% aufweist. Vorzugsweise enthält das erhaltene Mahlgut 50 Gew.-% an Mehlpartikeln mit einer Korngröße von mehr als 100 µm. In der Regel weisen mindestens 95 Gew.-% der vermahlenen Mehlpartikel eine Korngröße von weniger als 2 mm auf. Die Messung der Korngröße erfolgt dabei mittels Siebanalyse unter Verwendung einer Vibrations-Analysenmaschine. Eine geringe Korngröße ist grundsätzlich zur Erzielung einer hohen Produktausbeute vorteilhaft. Eine zu geringe Teilchengröße kann jedoch zu Problemen, insbesondere durch Klumpenbildung/Agglomeration, beim Anmaischen des Mahlguts während der Verflüssigung bzw. bei der Aufarbeitung, z. B. bei der Trocknung der Feststoffe nach dem Fermentationsschritt, führen.

Üblicherweise werden Mehle durch den Ausmahlungsgrad bzw. durch die Mehltype charakterisiert, wobei diese so miteinander korrelieren, dass mit zunehmendem Ausmahlungsgrad auch die Kennzahl der Mehltype zunimmt. Der Ausmahlungsgrad entspricht der Gewichtsmenge des gewonnenen Mehls bezogen auf 100 Gewichtsteile des eingesetzten Mahlguts. Während beim Mahlen zunächst reines, feinstes Mehl, z. B. aus dem Inneren des Getreidekorns, anfällt, nimmt beim weiteren Vermahlen, also mit steigendem Ausmahlungsgrad, der Anteil an Rohfaser- und Schalengehalt im Mehl zu, der Stärkeanteil wird dabei geringer. Der Ausmahlungsgrad spiegelt sich daher auch in der so genannten Mehltype wider, die als Zahlenangabe zur Klassifizierung von Mehlen, insbesondere von Getreidemehlen, verwendet wird und die auf dem Aschegehalt des Mehles beruht (so genannte Ascheskala). Die Mehltype bzw. die Typenzahl gibt hierbei die Menge Asche (Mineralstoffe) in mg an, die beim Verbrennen von 100 g Mehltrockensubstanz zurück bleibt. Für Getreidemehle bedeutet eine höhere Typzahl einen höheren Ausmahlungsgrad, da der Kern des Getreidekorns in etwa 0,4 Gew.-%, die Schale hingegen etwa 5 Gew.-% Asche enthält. Bei niederem Ausmahlungsgrad bestehen die Getreidemehle also überwiegend aus dem zerkleinerten Mehlkörper, d. h. dem Stärkebestandteil der Getreidekörner; bei höherem Ausmahlungsgrad enthalten die Getreidemehle auch die zerkleinerte, eiweißhaltige Aleuronschicht der Getreidekörner, bei Schrot auch die Bestandteile des eiweiß- und fetthaltigen Keimlings sowie der rohfaser- und aschehaltigen Samenschalen. Für die erfindungsgemäßen Zwecke sind grundsätzlich Mehle mit einem hohen Ausmahlungsgrad bzw. einer hohen Typzahl bevorzugt. Wird Getreide als Stärkequelle eingesetzt, so werden vorzugsweise die ganzen ungeschälten Körner vermahlen und weiter verarbeitet, gegebenenfalls nach vorheriger mechanischer Abtrennung von Keim und Spelzen.

Erfindungsgemäß enthält das verwendete Mahlgut zumindest einen Teil, vorzugsweise wenigstens 20 Gew.-%, insbesondere wenigstens 50 Gew.-%, speziell wenigstens 90 Gew.-% und ganz speziell wenigstens 99 Gew.-% der in den vermahlenen Getreidekörnern enthaltenen nicht-stärkehaltigen festen Bestandteile, entsprechend dem Ausmahlungsgrad. Bezogen auf die stärkehaltigen Bestandteile des Mahlguts (und damit auf die Menge an verstoffwechselbarem Zucker im dextrinhaltigen Medium (1)) beträgt der Anteil der nicht-stärkehaltigen festen Bestandteile im Mahlgut vorzugsweise wenigstens 10 Gew.-% und insbesondere wenigstens 15 Gew.-%, z. B. im Bereich von 15 bis 75 Gew.-% und speziell im Bereich von 20 bis 60 Gew.-%.

Das zur Verflüssigung vorgesehene Mahlgut wird in Schritt a2) erfindungsgemäß mit einer wässrigen Flüssigkeit, z. B. Frischwasser, rückgeführtem Prozesswasser, z. B. aus einer nachfolgenden Fermentation, oder mit einer Mischung dieser Flüssigkeiten vermischt. Hierbei wird man in der Regel eine wässrige Suspension erhalten. In der Regel wird man eine solche Menge der Stärkequelle bzw. des Mahlguts mit der wässrigen Flüssigkeit vermischen und in dieser verflüssigen, dass die erhaltene wässrige Dextrin-haltige Flüssigkeit (1) eine Konzentration an Glucoseäquivalenten von mindestens 40 Gew.-%, bezogen auf das Gesamtgewicht des Mediums (1), aufweist. Der Trockenmassegehalt in dem so erhaltenen Medium (1) beträgt typischerweise mindestens 50 Gew.-%, bezogen auf das Gesamtgewicht des Mediums (1).

Zur Durchführung des erfindungsgemäßen Verfahrens ist es möglich, die zum Suspendieren des festen Mahlguts verwendete wässrige Flüssigkeit auf eine leicht erhöht Temperatur, z. B. im Bereich von 40 bis 60 °C, vorzutemperieren. Es ist jedoch bevorzugt, die Flüssigkeiten bei Raumtemperatur einzusetzen.

Zur Durchführung der Verflüssigung des Stärkeanteils im Mahlgut gemäß Schritt a2) hat es sich als vorteilhaft erwiesen, vor dem Beginn der Verflüssigung nur eine Teilmenge des gesamten Mahlguts mit der wässrigen Flüssigkeit zu vermischen und das restliche Mahlgut erst im Verlauf der Verflüssigung kontinuierlich oder diskontinuierlich zu der wässrigen Flüssigkeit zu geben.

Das Verflüssigen des Mahlguts gemäß Schritt a2) kann nach üblichen, dem Fachmann bekannten Verfahren erfolgen, z. B. nach den in dem eingangs zitierten "The Alcohol Textbook - A reference for the beverage, fuel and industrial alcohol industries", Kapitel 2, S. 7 bis 23, beschriebenen Methoden.

Erfindungsgemäß erfolgt das Verflüssigen in Schritt a2) in Gegenwart mindestens eines Stärke verflüssigenden Enzyms. Hierzu können grundsätzlich alle Stärke verflüssigenden Enzyme, insbesondere α-Amylasen (Enzymklasse EC 3.2.1.1) eingesetzt werden, beispielsweise α-Amylasen, die aus *Bacillus lichenformis* oder *Bacillus staerothermophilus* gewonnen wurden und speziell solche, die zum Verflüssigen von durch Dry-Milling-Verfahren gewonnenen Materialien im Rahmen der Herstellung von Bioethanol verwendet werden. Die zum Verflüssigen geeigneten α-Amylasen sind auch kommerziell erhältlich, beispielsweise von Novozymes unter der Bezeichnung Termamyl 120 L, Typ L; oder von Genencor unter der Bezeichnung Spezyme. Es kann auch eine Kombination verschiedener α-Amylasen zur Verflüssigung eingesetzt werden.

Hierbei erhält man eine wässrige Flüssigkeit, welche den verflüssigten Stärkeanteil aus dem Mahlgut, typischerweise Dextrine mit in der Regel 3 bis 18, insbesondere 6 bis 12 Monosaccharid-Einheiten, gegebenenfalls weitere Oligosaccharide, gegebenenfallls geringe Mengen an Mono- und/oder Disacchariden (in der Regel < 30 Gew.-%, häufig < 25 Gew.-%, < 20 Gew.-%, insbesondere < 10 Gew.-%, bezogen auf die Gesamtmenge an Mono-, Di- und Oligosacchariden) sowie die nicht-stärkehaltigen Bestandteile des eingesetzten Mahlguts, insbesondere die festen, nicht-stärkehaltigen Bestandteile des zur Verflüssigung eingesetzten Mahlguts enthält.

Vorteilhafterweise werden die Mengen an Stärke verflüssigendem Enzym und Mahlgut, so gewählt sein, dass die Viskosität während des Gelierungsvorgangs ausreichend reduziert ist, um eine effektive Durchmischung der Suspension, z. B. mittels Rühren, zu ermöglichen. Bevorzugt beträgt die Viskosität der Reaktionsmischung während des Gelierens maximal 20 Pas, besonders bevorzugt maximal 15 Pas und ganz besonders bevorzugt maximal 8 Pas. Die Messung der Viskosität erfolgt in der Regel mit einem Haake Viskosimeter Type Roto Visko RV20 mit Messsystem M5 und Messeinrichtung MVDIN bei einer Temperatur von 50 °C und einer Schergeschwindigkeit von 200 s⁻¹.

Die α-Amylase (bzw. das verwendete Stärke verflüssigende Enzym) kann im Reaktionsgefäß vorgelegt oder im Verlauf des Schrittes a2) zugegeben werden. Vorzugsweise gibt man eine Teilmenge der in Schritt a2) benötigten α-Amylase zu, Beginn von Schritt a2) zu oder legt diese Teilmenge im Reaktor vor. Die Gesamtmenge an α-Amylase liegt üblicherweise im Bereich von 0,002 bis 3,0 Gew.-%, bevorzugt von 0,01 bis 1,5 Gew.-%, und besonders bevorzugt von 0,02 bis 0,5 Gew.-%, bezogen auf die Gesamtmenge der eingesetzten Stärkequelle.

Die Verflüssigung kann oberhalb oder unterhalb der Gelierungstemperatur durchgeführt werden. Vorzugsweise erfolgt die Verflüssigung in Schritt a2) zumindest zeitweise oberhalb der Gelierungstemperatur bzw. Verkleisterungstemperatur der eingesetzten Stärke (so genannter Cooking Prozess). Die hierzu für die jeweilige Stärke erforderliche Temperatur ist dem Fachmann bekannt (siehe das eingangs zitierte "The Alcohol Textbook - A reference for the beverage, fuel and industrial alcohol industries", Kapitel 2, S. 11) oder kann von ihm in Routineexperimenten bestimmt werden. In der Regel wird eine Temperatur im Bereich von 80 bis 165 °C, bevorzugt im Bereich von 90 bis 150 °C und besonders bevorzugt im Bereich von 100 bis 140 °C gewählt, wobei die Temperatur in der Regel wenigstens 5 K, vorzugsweise mindestens 10 K, und besonders bevorzugt mindestens 20 K, z. B. 10 bis 100 K, insbesondere 20 bis 80 K oberhalb der Gelierungstemperatur liegt. Bei diesen Temperaturen wird die granuläre Struktur der Stärke zerstört (Gelieren), wodurch deren enzymatischer Abbau ermöglicht wird.

Für eine optimale Wirkung der α-Amylase (bzw. des verwendeten Stärke verflüssigenden Enzyms) wird Schritt a2) vorzugsweise zumindest zeitweise im pH-Optimum des verflüssigenden Enzyms, häufig bei einem pH-Wert im schwach sauren Bereich, vorzugsweise zwischen 4,0 und 7,0, besonders bevorzugt zwischen 5,0 bis 6,5 durchgeführt, wobei üblicherweise vor oder zu Beginn von Schritt a2) die pH-Einstellung vorgenommen wird; dieser pH-Wert wird während der Verflüssigung vorzugsweise kontrolliert und gegebenenfalls nachgestellt. Die Einstellung des pH-Werts erfolgt vorzugsweise mit verdünnten Mineralsäuren wie H₂SO₄ oder H₃PO₄ bzw. mit verdünnten Alkalilaugen wie NaOH oder KOH.

In einer bevorzugten Ausführungsform gibt man zum Verflüssigen des Stärkeanteils im Mahlgut gemäß Schritt a2) mindestens eine Teilmenge des Mahlguts kontinuierlich oder diskontinuierlich zu der wässrigen Flüssigkeit. Vorzugsweise gibt man hierbei wenigstens 40 Gew.-%, insbesondere wenigstens 50 Gew.-% und ganz besonders bevorzugt wenigstens 55 Gew.-% im Verlauf der Verflüssigung in den Reaktor. Häufig wird die zugegebene Menge 90 Gew.-%, insbesondere 85 Gew.-% und besonders bevorzugt 80 Gew.-% nicht überschreiten. Vorzugsweise wird die im Verlauf zugegebene Teilmenge an Mahlgut dem Reaktor unter Bedingungen zugeführt, wie sie bei der Verflüssigung vorliegen. Die Zugabe kann diskontinuierlich, d. h. portionsweise in mehreren Teilportionen, die vorzugsweise jeweils nicht mehr als 30 Gew.-%, besonders bevorzugt nicht mehr als 20 Gew.-%, z. B. 1 bis 30 Gew.-% und insbesondere 2 bis 20 Gew.-%, der Gesamtmenge des zu verflüssigenden Mahlgutes ausmachen, oder kontinuierlich erfolgen. Wesentlich bei dieser Ausführungsform ist, dass sich zu Beginn der Verflüssigung nur ein Teil des Mahlgutes, vorzugsweise nicht mehr als 60 Gew.-%, insbesondere nicht mehr als 50 Gew.-% und besonders bevorzugt nicht mehr als 45 Gew.-% des Mahlgutes, im Reaktor befindet und die Restmenge des Mahlgutes während des Verflüssigens zugegeben wird.

Die Verflüssigung kann auch kontinuierlich, z. B. in einer mehrstufigen Reaktionskaskade, durchgeführt werden.

In einer bevorzugten Ausführungsform führt man Schritt a2) des erfindungsgemäßen Verfahrens so durch, dass zunächst eine Teilmenge von höchstens 60 Gew.-%, bevorzugt höchstens 50 Gew.-% und besonders bevorzugt höchstens 45 Gew.-%, z. B. 10 bis 60 Gew.-%, insbesondere 15 bis 50 Gew.-% und besonders bevorzugt 20 bis 45 Gew.-%, bezogen auf die Gesamtmenge des Mahlguts, in der wässrigen Flüssigkeit, suspendiert wird und anschließend die Verflüssigung durchgeführt wird.

In einer bevorzugten Ausführungsform erfolgt die diskontinuierliche oder kontinuierliche, insbesondere portionsweise Zugabe einer Teilmenge des Mahlguts in Gegenwart der mindestens einen α-Amylase derart, dass die Viskosität des Flüssigmediums maximal 20 Pas, bevorzugt maximal 15 Pas und besonders bevorzugt maximal 8 Pas beträgt. Zur Unterstützung der Viskositätskontrolle hat es sich als vorteilhaft erwiesen, wenn mindestens 25 Gew.-%, bevorzugt mindestens 35 Gew.-% und besonders bevorzugt mindestens 50 Gew.-% der Gesamtmenge des zugegebenen Mahlguts bei einer Temperatur oberhalb der Gelatinierungstemperatur der im Mahlgut enthaltenen Stärke zugegeben werden. Die Kontrolle der Viskosität kann des Weiteren dadurch beeinflusst werden, dass das mindestens eine Stärke verflüssigende Enzym, vorzugsweise eine α-Amylase, oder/und das mindestens eine verzuckernde Enzym, vorzugsweise eine Glucoamylase, selbst portionsweise zugegeben werden.

Zur Durchführung des erfindungsgemäßen Verfahrens ist es möglich, die zum Suspendieren des festen Mahlguts verwendete wässrige Flüssigkeit auf eine leicht erhöhte Temperatur, z. B. im Bereich von 40 bis 60 °C, vorzutemperieren. Es ist jedoch bevorzugt, die Flüssigkeiten bei Raumtemperatur einzusetzen.

Zu der Suspension des Mahlguts wird dann das mindestens eine Stärke verflüssigende Enzym, vorzugsweise eine α-Amylase, gegeben. Wird eine Teilmenge des Mahlguts erst im Verlauf der Verflüssigung hinzugegeben, so gibt man anfangs vorteilhafterweise nur eine Teilmenge der α-Amylase zu, z. B. 10 bis 70 Gew.-% und insbesondere 20 bis 65 Gew.-%, bezogen auf die insgesamt in Schritt a2) eingesetzte α-Amylase. Die zu diesem Zeitpunkt zugegebene Menge an α-Amylase richtet sich in diesem Fall nach der Aktivität der jeweiligen α-Amylase in Bezug auf die verwendete Stärkequelle unter den Reaktionsbedingungen und liegt üblicherweise im Bereich von 0,0004 bis 2,0 Gew.-%, bevorzugt von 0,001 bis 1,0 Gew.-% und besonders bevorzugt von 0,02 bis 0,3 Gew.-%, bezogen auf die Gesamtmenge der eingesetzten Stärkequelle. Alternativ kann hierbei die Teilmenge der α-Amylase vor dem Ansetzen der Suspension mit der verwendeten Flüssigkeit vermengt werden.

Vorzugsweise wird die eingesetzte Menge oder Teilmenge an α-Amylase vor dem Beginn des Aufheizens auf die zur Verflüssigung angewendete Temperatur, insbesondere bei Raumtemperatur oder nur leicht erhöhter Temperatur, z. B. im Bereich von 20 bis 30 °C, zu der Suspension gegeben.

Die so angesetzte Suspension wird dann vorzugsweise auf eine Temperatur oberhalb der Gelierungstemperatur der verwendeten Stärke erhitzt. In der Regel wird eine Temperatur im Bereich von 80 bis 165 °C, bevorzugt im Bereich von 90 bis 150 °C, und besonders bevorzugt im Bereich von 100 bis 140 °C gewählt, wobei die Temperatur in der Regel wenigstens 5 K, vorzugsweise mindestens 10 K und besonders bevorzugt mindestens 20 K, z. B. 10 bis 100 K, insbesondere 20 bis 80 K oberhalb der Gelierungstemperatur liegt. Unter Kontrolle der Viskosität werden gegebenenfalls nach und nach weitere Teilmengen der Stärkequelle, z. B. jeweils 1 bis 30 Gew.-% und insbesondere 2 bis 20 Gew.-%, bezogen auf die gesamte eingesetzte Menge an Mahlgut, zu der stärkehaltigen Suspension gegeben. In diesem Fall ist es bevorzugt, die im Verlauf der Verflüssigung zuzugebende Teilmenge des Mahlgutes in mindestens 2, bevorzugt mindestens 4 und besonders bevorzugt mindestens 6 Teilportionen der Reaktionsmischung zuzugeben. Alternativ kann in dieser Ausführungsform die Zugabe der beim Ansetzen der Suspension nicht eingesetzten Teilmenge des Mahlgutes kontinuierlich während der Verflüssigung erfolgen. Die Temperatur sollte bei der Zugabe vorteilhafterweise oberhalb der Gelierungstemperatur der Stärke gehalten werden.

Nach Erreichen der gewünschten Temperatur bzw. gegebenenfalls nach vollständiger Zugabe des Mehls wird das Reaktionsgemisch üblicherweise noch eine Zeit, z. B. 10 bis 60 Minuten oder länger, sofern erforderlich, bei der oberhalb der Gelierungstemperatur der Stärke eingestellten Temperatur gehalten, d. h. verkocht. Die Reaktionsmischung wird dann in der Regel auf eine etwas geringere Temperatur, jedoch vorzugsweise oberhalb der Gelierungstemperatur, z. B. auf 70 bis 90 °C, abgekühlt. Anschließend wird gegebenenfalls eine weitere Teilmenge an α-Amylase, vorzugsweise die Hauptmenge, zugesetzt. In diesem Fall beträgt die Menge an zu diesem Zeitpunkt zugegebener α-Amylase je nach Aktivität der verwendeten α-Amylase unter den Reaktionsbedingungen vorzugsweise 0,002 bis 2,0 Gew.-%, besonders bevorzugt von 0,01 bis 1,0 Gew.-% und ganz besonders bevorzugt von 0,02 bis 0,4 Gew.-%, bezogen auf die Gesamtmenge der eingesetzten Stärkequelle.

Zum vollständigen Abbau der Stärke zu Dextrinen wird das Reaktionsgemisch so lange bei der eingestellten Temperatur gehalten oder gegebenenfalls weiter erhitzt, bis der Stärkenachweis mit Jod oder gegebenenfalls ein anderer Test zum Nachweis von Stärke negativ oder mindestens im Wesentlichen negativ ausfällt. Gegebenenfalls können hierbei noch eine oder mehrere weitere Teilmengen α-Amylase, z. B. im Bereich von 0,001 bis 0,5 Gew.-% und bevorzugt 0,002 bis 0,2 Gew.-%, bezogen auf die Gesamtmenge der eingesetzten Stärkequelle, zu der Reaktionsmischung gegeben werden.

Alternativ kann man die das Mahlgut enthaltende wässrige Suspension zur Verflüssigung des Stärkeanteils zunächst durch Einbringen von Wasserdampf auf eine Temperatur oberhalb der Verkleisterungstemperatur der in der Stärkequelle bzw. dem Mahlgut enthaltenen Stärke erhitzen. Typischerweise wird man auf eine Temperatur erhitzen, die wenigstens 10 K und insbesondere wenigstens 20 K, z. B. 10 bis 100 K, insbesondere 20 bis 80 K, oberhalb der jeweiligen Verkleisterungstemperatur liegt. Insbesondere erhitzt man die Suspension auf Temperaturen im Bereich von 90 bis 150 °C, und speziell im Bereich von 100 bis 140 °C.

Bei dem zum Erhitzen eingesetzten Wasserdampf handelt es sich typischerweise um überhitzen Wasserdampf, der eine Temperatur von wenigstens 105 °C, insbesondere wenigstens 110 °C, z. B. 110 bis 210 °C aufweist. Vorzugsweise wird der Dampf mit

Überdruck in die Suspension eingebracht. Dementsprechend weist der Dampf vorzugsweise einen Druck von wenigstens 1,5 bar, z. B. 1,5 bis 16 bar, insbesondere 2 bis 12 bar auf.

Das Einbringen von Wasserdampf in die Suspension erfolgt in der Regel so, dass man den Dampf mit Überdruck, vorzugsweise einem Überdruck von 1 bis 10 oder 11 bar, insbesondere 1,5 bis 5 bar und vorzugsweise mit hoher Geschwindigkeit in die Suspension einträgt. Durch das Eintragen des Dampfes erhitzt sich die Suspension augenblicklich auf Temperaturen oberhalb 90 °C, also auf Temperaturen oberhalb der Verkleisterungstemperatur.

Vorzugsweise erfolgt das Erhitzen mit Wasserdampf in einer kontinuierlich arbeitenden Vorrichtung, in welche man die Suspension kontinuierlich mit einem bestimmten Förderdruck, der sich aus der Viskosität der Suspension, der Fördergeschwindigkeit und der Geometrie der Vorrichtung ergibt, einspeist und in die man im Bereich des Einspeisens der Suspension den heißen Dampf mit Überdruck, bezogen auf den Förderdruck, über eine regelbare Düse einspeist. Durch das Einspeisen des Dampfs mit Überdruck wird die Suspension nicht nur erhitzt sondern es wird auch mechanische Energie in das System eingetragen, welche eine weitere Zerteilung der Mahlgutpartikel fördert, einen besonders gleichmäßigen Energieeintrag bewirkt, und somit eine besonders gleichmäßige Verkleisterung der granulären Stärkepartikel im Mahlgut zur Folge hat. Typischerweise haben diese Vorrichtungen eine röhrenförmige Geometrie. Vorzugsweise erfolgt das Eintragen des Dampfes in Richtung der Längsachse der röhrenförmigen Vorrichtung. Die Zufuhr der Suspension erfolgt in der Regel in einem Winkel von wenigstens 45° oder senkrecht hierzu. Die regelbare Düse weist typischerweise eine konische Geometrie auf, die sich in Fließrichtung des Dampfs verjüngt. In dieser Düse ist eine Nadel oder ein auf einer in Längsrichtung verschiebbaren Stange angeordneter Kegel angeordnet. Nadel bzw. Kegel bildet mit dem Konus der Düse einen Spalt. Durch Verschieben der Nadel bzw. der Stange in Längsrichtung lässt sich die Größe des Spalts und damit die Querschnittsfläche der Düsenöffnung in einfacher Weise einstellen, wodurch man in einfacher Weise die Geschwindigkeit des Dampfeintrags regulieren kann.

Typischerweise weisen diese Vorrichtungen außerdem ein Vermischungsrohr auf, in welches die Suspension nach dem Dampfeintrag transportiert und aus der Vorrichtung ausgetragen wird. Dieses Vermischungsrohr ist üblicherweise in Richtung des Dampfeintrags und senkrecht zur Einspeisung angeordnet. Das Vermischungsrohr bildet typischerweise mit der Düse einen Spalt, durch den die Suspension transportiert wird. Durch diesen Spalt wirken beim Transport zusätzliche Scherkräfte auf die Suspension und erhöhen somit den mechanischen Energieeintrag in die Suspension. Das Vermischungsrohr kann in Längsrichtung verschiebbar angeordnet sein. Durch Verschieben des Vermischungsrohrs lässt sich in einfacher Weise die Größe der Spaltöffnung einstellen und damit das Druckgefälle in der Vorrichtung.

Derartige Vorrichtungen sind unter der Bezeichnung Jet-Kocher aus dem Stand der Technik bekannt, beispielsweise die in "The Alcohol Textbook", Kapitel 2, loc. cit., Figur 13 dargestellte Vorrichtung und kommerziell erhältlich, beispielsweise unter der Bezeichnung HYDROHEATER® der Fa. Hydro Thermal Corp. Waukesha WI, USA.

Bei kontinuierlicher Reaktionsführung wird die mit Wasserdampf behandelte Suspension in der Regel im Anschluss daran in eine Nachreaktionszone überführt, um das Gelieren der Stärkebestandteile fortzusetzen. In der Nachreaktionszone herrscht typischerweise Überdruck, typischerweise ein Absolutdruck im Bereich von 2 bis 8 bar. Die Temperaturen in der Nachreaktionszone liegen typischerweise im Bereich von 90 bis 150 °C. Die Verweilzeit in dieser Nachreaktionszone kann in Abhängigkeit von der Temperatur der Suspension im Bereich von 1 min bis 4 h betragen. Die Nachreaktionszonen weisen typischerweise eine röhrenförmige oder säulenförmige Geometrie auf. In einer Ausführungsform handelt weist die Nachreaktionszone die Geometrie einer senkrecht angeordneten Säule auf. Die Suspension wird hierbei nach Verlassen der Vorrichtung zur Dampfbehandlung im oberen Bereich der Säule aufgebracht und im unteren Bereich entnommen. In einer anderen Ausführungsform der Erfindung weist die Nachreaktionszone eine röhrenförmige Geometrie auf.

Nach Verlassen der Nachreaktionszone wird die Suspension in der Regel entspannt und man führt dann eine Verflüssigung durch. Vorzugsweise führt man die Entspannung als Flash-Verdampfung durch, um die Suspension abzukühlen, vorzugsweise auf Temperaturen unterhalb 100 °C, insbesondere unterhalb 85 °C. In der Regel erfolgt dann eine Verflüssigung der so aufgeschlossenen Stärke in einem separaten Reaktionsgefäß. Die Verflüssigung kann in der oben beschriebenen Weise durchgeführt werden.

In einer bevorzugten Ausführungsform der Erfindung gibt man wenigstens einen Teil oder die Gesamtmenge, in der Regel wenigstens 50 %, insbesondere wenigstens 80 % der Gesamtmenge oder die Gesamtmenge des die Stärke verflüssigenden Enzyms vor dem Erhitzen mit Wasserdampf zu der Suspension des Mahlguts in der wässrigen Flüssigkeit. Auf diese Weise erfolgt die Verflüssigung bereits während des Erhitzens auf Temperaturen oberhalb der Verkleisterungstemperatur. Das Erhitzen mit Wasserdampf und die Nachreaktionsphase werden entsprechend durchgeführt. Eine anschließende Verflüssigung in einem separaten Reaktionsgefäß kann entfallen. Vorzugsweise wird man jedoch eine solche Verflüssigung zurVervollständigung des Abbaus der Stärke in Dextrine durchführen.

Zur Stabilisierung der eingesetzten Enzyme kann gegebenenfalls die Konzentration an Ca²⁺-lonen, z. B. mit CaCl₂, auf einen enzymspezifischen optimalen Wert eingestellt werden. Geeignete Konzentrationswerte können vom Fachmann in Routineexperimenten bestimmt werden. Wird z. B. Termamyl als α-Amylase eingesetzt, so ist es vorteilhaft, eine Ca²⁺-Konzentration von z. B. 10 bis 100 ppm, bevorzugt 20 bis 80 ppm und besonders bevorzugt etwa 30 bis 70 ppm im Flüssigmedium einzustellen, wobei die Angabe ppm gewichtsbezogen ist und g/1000kg bedeutet.

Zum vollständigen Abbau der Stärke zu Dextrinen wird das Reaktionsgemisch so lange bei der eingestellten Temperatur gehalten, bis der Stärkenachweis mit Jod oder gegebenenfalls ein anderer Test zum Nachweis von Stärke negativ oder mindestens im Wesentlichen negativ ausfällt. Gegebenenfalls können hierbei noch eine oder mehrere weitere Teilmengen α-Amylase, z. B. im Bereich von 0,001 bis 0,5 Gew.-% und bevorzugt 0,002 bis 0,2 Gew.-%, bezogen auf die Gesamtmenge der eingesetzten Stärkequelle, zu der Reaktionsmischung gegeben werden.

Da zur Herstellung der Dextrin-haltigen Flüssigkeit (1) in der Regel Mahlgut eingesetzt wird, welches im Wesentlichen alle oder nahezu alle Bestandteile der Stärkequelle, oder zumindest neben der Stärke auch einen Teil der festen nicht-stärkehaltigen Bestandteile enthält (d. h. es wird keine vollständige Abtrennung der nicht-stärkehaltigen festen Bestandteile der Stärkequelle vorgenommen), umfasst die erhaltene Dextrin-haltige Flüssigkeit (1) auch einen Teil oder die Gesamtmenge der nicht-stärkehaltigen festen Bestandteile der Stärkequelle. Dies bedingt oftmals den Eintrag eines nicht zu vernachlässigenden Anteils an Phytat, z. B. aus der Kornfrucht. Um die daraus resultierende inhibierende Wirkung zu vermeiden, wird vorteilhafterweise in Schritt a2) dem Medium (1) mindestens eine Phytase zugesetzt, bevor es einem Fermentationsschritt zugeführt wird. Die Zugabe der Phytase kann vor, während oder nach der Verflüssigung erfolgen, sofern sie die jeweils erforderliche Hitzestabilität aufweist. Es können beliebige Phytasen eingesetzt werden, soweit deren Aktivität unter den Reaktionsbedingungen jeweils höchstens unwesentlich eingeschränkt ist. Bevorzugt sind Phytasen mit einer Temperaturstabilität (T50) > 50 °C und besonders bevorzugt > 60 °C. Die Menge an Phytase beträgt üblicherweise 1 bis 10000 Units/kg Stärkequelle und insbesondere 10 bis 4000 Units/kg Stärkequelle.

Es hat sich als vorteilhaft erwiesen, während der Herstellung der Dextrin-haltigen Flüssigkeit (1) außerdem weitere Enzyme, zum Beispiel Pullulanasen, Cellulasen, Hemicellulasen, Glucanasen, Xylanasen oder Proteasen, zuzusetzen. Der Zusatz dieser Enzyme kann die Viskosität positiv beeinflussen, d. h. herabsetzen (z. B. durch Spaltung langkettiger (auch als längerkettiger bezeichnet) Glucane und/oder von (Arabino)Xylanen), die Freisetzung metabolisierbarer Glucoside und die Freisetzung von (Rest)Stärke bewirken. Der Einsatz von Proteasen hat analoge positive Effekte, wobei zusätzlich Aminosäuren als Wachstumsfaktoren für die Fermentation freigesetzt werden können.

Die in Schritt a2) erhaltene Dextrin-haltige Flüssigkeit (1) weist in der Regel eine Konzentration an Glucoseäquivalenten von mindestens 20 Gew.-% (= 200 g/kg), insbesondere mindestens 40 Gew.-% und speziell mindestens 50 Gew.-%, häufig im Bereich von 30 bis 75 Gew.-%, vorzugsweise im Bereich von 40 bis 70 Gew.-%, insbesondere im Bereich von 50 bis 65 Gew.-%, jeweils bezogen auf das Gesamtgewicht des Mediums (1), auf.

Der Trockenmassegehalt in der so erhaltenen Flüssigkeit (1) beträgt typischerweise mindestens 25 Gew.-%, vorzugsweise mindestens 40 Gew.-%, insbesondere mindestens 50 Gew.-%, speziell mindestens 60 Gew.-%, und wird in der Regel 80 Gew.-%, jeweils bezogen auf das Gesamtgewicht des Mediums (1), nicht überschreiten.

Die in der so erhaltenen Dextrin-haltigen Flüssigkeit (1) enthaltenen Glucoseäquivalente liegen im Wesentlichen in Form von Oligosacchariden, insbesondere Dextrinen vor. Hauptbestandteil dieser Oligosaccharide bzw. Dextrine ist typischerweise Glucose, wobei das Medium auch geringe Mengen an Mono- und/oder Disacchariden und aus anderen Monosaccharid-Einheiten aufgebaute Oligosaccharid-Einheiten enthalten kann. Typischerweise umfassen die zuckerhaltigen Bestandteile im Dextrin-haltigen Medium (1), d. h. die Mono-, Di- und Oligosaccharide, einen Anteil an Oligosacchariden, insbesondere Dextrinen von mindestens 70 Gew.-%, häufig mindestens 75 Gew.-% insbesondere mindestens 80 Gew.-%, speziell mindestens 90 Gew.-%, d. h. der Anteil der Mono- und Disaccharide ist weniger als 30 Gew.-%, häufig weniger als 25 Gew.-%, insbesondere weniger als 20 Gew.-% und speziell weniger als 10 Gew.-%. Üblicherweise liegt der Anteil an Glucose, in freier oder gebundener Form, unter den Glucoseäquivalenten des Mediums (1) im Bereich von 50 bis 99 Gew.-%, insbesondere von 75 bis 97 Gew.-% und speziell von 80 bis 95 Gew.-%, bezogen auf die gesamte Menge an Glucoseäquivalenten.

Die in Schritt a2) erhaltene wässrige Dextrin-haltige Flüssigkeit (1) wird erfindungsgemäß in Schritt b) zur fermentativen Herstellung der gewünschten organischen Verbindung verwendet. Hierzu wird die Dextrin-haltige Flüssigkeit (1) einer Fermentation zugeführt, wo es zur Kultivierung der in der Fermentation eingesetzten Mikroorganismen dient. Die jeweilige organische Verbindung wird hierbei als flüchtiges oder nichtflüchtiges mikrobielles Stoffwechselprodukt erhalten.

In der Regel wird die erhaltene wässrige Dextrin-haltige Flüssigkeit (1) unter Wegfall eines separaten Verzuckerungstanks direkt in einer Fermentation gemäß Schritt b) eingesetzt. In der Regel wird man die Dextrin-haltige Flüssigkeit (1) auf Fermentationstemperatur, üblicherweise im Bereich von 32 bis 37 °C, abkühlen, bevor man es der Fermentation zuführt.

Die wässrige Dextrin-haltige Flüssigkeit (1) kann vor der Fermentation gegebenenfalls sterilisiert werden, wobei man die Mikroorganismen üblicherweise durch thermische oder chemische Verfahren abtötet. Beispielsweise heizt man hierzu die wässrige Dextrin-haltige Flüssigkeit (1) auf Temperaturen von üblicherweise oberhalb 80 °C auf. Die Abtötung bzw. Lyse der Zellen kann unmittelbar vor der Fermentation erfolgen. Hierzu wird die gesamte Dextrin-haltige Flüssigkeit (1) der Lyse bzw. Abtötung zugeführt. Diese kann thermisch, mechanisch oder chemisch erfolgen. Es hat sich jedoch im Rahmen des erfindungsgemäßen Verfahrens als nicht notwendig erwiesen, vor der Fermentation einen Sterilisierungsschritt wie hier beschrieben durchzuführen, sondern es hat sich vielmehr als vorteilhaft erwiesen, keinen solchen Sterilisierungsschritt durchzuführen. Dementsprechend betrifft eine bevorzugte Ausführungsform der Erfindung ein Verfahren, bei dem das in Schritt a2) erhaltene Medium (1) unmittelbar, d. h. ohne vorherige Sterilisation, der Fermentation zugeführt wird.

Bei der Fermentation erfolgt die Verstoffwechselung der Dextrine erfindungsgemäß im Wesentlichen ohne Zugabe verzuckernder Enzyme. Hierbei werden die Dextrine von dem Mikroorganismus verstoffwechselt, vermutlich nachdem sie durch Stamm-eigene verzuckernde Enzyme, z. B. Stamm-eigene Glucoamylasen, hydrolysiert wurden. Vermutlich erfolgt eine Verzuckerung der verflüssigten Stärkebestandteile parallel zur Verstoffwechselung des Zuckers, insbesondere des Monosaccharids Glucose, durch die Mikroorganismen.

Daher ist der zur Fermentation eingesetzte Mikroorganismus unter Mikroorganismen ausgewählt, die Enzyme exprimieren bzw. produzieren, welche Dextrine zu Monosacchariden hydrolysieren, insbesondere unter solchen, die Glucoamylasen produzieren bzw. exprimieren. Derartige Mikroorganismen sind dem Fachmann bekannt, oder können durch Routineexperimente, z. B. durch Screeningverfahren, beispielsweise durch ein Screening auf Glucoamylase, z. B. durch Anziehen des Mikroorganismus im Schüttelkolbentest mit anschließendem Enzymaktivitätstest auf Glucoamylase, oder durch Screening mit Hilfe von Primern/Sonden nach den im Beispielteil beschriebenen Screeningmethoden, sowie über Datenbankrecherchen in Enzymdatenbanken wie
- Brenda [Schomburg I., Chang A., Hofmann O., Ebeling C., Ehrentreich F., Schomburg D. BRENDA: a resource for enzyme data and metabolic information. Trends Biochem Sci. 2002 Jan;27(1):54-6.],
- Swissprot [Boeckmann B., Bairoch A., Apweiler R., Blatter M.-C., Estreicher A., Gasteiger E., Martin M.J., Michoud K., O'Donovan C., Phan I., Pilbout S., Schneider M. *The SWISS-PROT protein knowledgebase and its supplement TrEMBL in 2003* Nucleic Acids Res. 31:365-370(2003)],
- ERGO-WIT [Overbeek R, Larsen N, Walunas T, D'Souza M, Pusch G, Selkov E Jr, Liolios K, Joukov V, Kaznadzey D, Anderson I, Bhattacharyya A, Burd H, Gardner W, Hanke P, Kapatral V, Mikhailova N, Vasieva O, Osterman A, Vonstein V, Fonstein M, Ivanova N, Kyrpides N. The ERGO(TM) genome analysis and discovery system. Nucleic Acids Res 2003 Jan 1;31(1): 164-71; Overbeek R, Larsen N, Pusch GD, D'Souza M, Selkov E Jr, Kyrpides N, Fonstein M, Maltsev N, Selkov E. WIT: integrated system for high-throughput genome sequence analysis and metabolic reconstruction. Nucleic Acids Research, 2000; Vol. 28, No.1: 123-125],
- CAZY [Coutinho, P.M. & Henrissat, B. (1999) Carbohydrate-Active Enzymes server at URL: http://afnib.cnrs-mrs.fr/CAZY/; Coutinho, P.M. & Henrissat, B. (1999) Carbohydrate-active enzymes: an integrated database approach. In "Recent Advances in Carbohydrate Bioengineering", H.J. Gilbert, G. Davies, B. Henrissat and B. Svensson eds., The Royal Society of Chemistry, Cambridge, pp. 3-12] und
- PIR [Cathy H. Wu, Lai-Su L. Yeh, Hongzhan Huang, Leslie Arminski, Jorge Castro-Alvear, Yongxing Chen, Zhang-Zhi Hu, Robert S. Ledley, Panagiotis Kourtesis, Baris E. Suzek, C. R. Vinayaka, Jian Zhang, and Winona C. Barker. The Protein Information Resource. Nucleic Acids Research, 31: 345-347, 2003.]
nach der im Beispielteil beschriebenen Methode ermittelt werden.

Beispiele für geeignete Mikroorganismen mit Glucoamylaseaktivität sind Agrobacterium tumefaciens, Arxula adeninivorans, Ashbya gossypii, Aspergillus awamori, Aspergillus candidus, Aspergillus foetidus, Aspergillus fumigatus, Aspergillus kawachi, Aspergillus nidulans, Aspergillus niger, Aspergillus oryzae, Aspergillus phoenicis, Aspergillus saitoi, Aspergillus shirousami, Aspergillus terreus, Athelia rolfsii, Bacillus circulans, Bacillus stearothermophilus, Beta vulgaris, Bradyrhizobium japonicum, Burkholderia cenocepacia, Burkholderia fungorum, Burkholderia pseudomallei, Candida albicans, Candida antarctica, Candida glabrata, Candida tsukubaensis, Caulobacter crescentus, Cephalosporium charticola, Cephalosporium eichhorniae Ceratocystis paradoxa , Chaetomium thermophilum , Chlorobium tepidum" Chromobacterium violaceum, Cladosporium resinae, Clostridium sp., Clostridium thermocellum, Clostridium thermosaccharolyticum, Coniophora puteana, Corticium rolfsii, Corynebacterium glutamicum, Cryptococcus neoformans, Debaryomyces hansenii, Debaryomyces occidentalis, Emericella nidulans, Endomyces sp., Endomycopsis fibuligera, Fusarium venenatum, Haloarcula marismortui, Hormoconis resinae, Humicola grisea, Humicola lanuginosa, Hypocrea lixii, Kluyveromyces lactis, Lentinula edodes, Lipomyces kononenkoae, Magnaporthe grisea, Mesorhizobium loti, Methanocaldococcus jannaschii, Methanococcus jannaschii, Methanococcus maripaludis, Methanosarcina acetivorans, Methanosarcina barkeri, Methanosarcina mazei, Monascus rubiginosus, Monascus sp., Mucor rouxianus, Mycobacterium bovis, Mycobacterium leprae, Mycobacterium marinum, Mycobacterium tuberculosis, Myrothecium sp., Neurospora crassa, Nostoc punctiforme, Oryza sativa, Paecilomyces variotii, Penaeus japonicus, Penicillium chrysogenum, Penicillium oxalicum, Picrophilus torridus, Pseudomonas fluorescens, Pseudomonas putida, Pseudomonas syringae, Ralstonia eutropha, Ralstonia metallidurans, Rana japonica, Rhizobium leguminosarum, Rhizopus delemar, Rhizopus javanicus, Rhizopus niveus, Rhizopus oryzae, Rhizopus sp., Rhodococcus sp., Rhodopseudomonas palustris, Rhodospirillum rubrum, Saccharomyces cerevisiae, Saccharomyces diastaticus, Saccharomycopsis fibuligera, Saccharomycopsis fibuligera, Schizosaccharomyces pombe, Schwanniomyces occidentalis, Shewanella oneidensis, Sphingomonas aromaticivorans, Streptomyces coelicolor, Sulfolobus acidocaldarius, Sulfolobus solfataricus, Talaromyces emersonii, Termitomyces clypeatus, Thermoactinomyces vulgaris, Thermoanaerobacter tengcongensis, Thermoanaerobacterium thermosaccharolyticum, Thermoascus crustaceus, Thermomyces lanuginosus, Thermoproteus tenax, Thielavia terrestris, Trichoderma reesei und Trichosporon adeninovorans.

Sofern die zur Fermentation eingesetzten Mikroorganismen Stamm-eigene Glucoamylasen exprimieren, kann der pH-Wert des Fermentationsmediums auf einen Wert im optimalen Wirkungsbereich für Glucoamylase eingestellt werden, z. B. auf einen Wert im Bereich zwischen 3,5 und 6,0. Häufig wird der pH-Wert auf einen für die Fermentation optimalen Wert einstellen, der außerhalb des genannten Bereichs liegen kann, z. B. im Bereich von 6,0 bis 8,0. Dies kann bei der Fermentation trotz der eingeschränkten Aktivität vieler Glucoamylasen in diesem pH-Bereich insgesamt vorteilhaft oder aufgrund der einzustellenden Fermentationsbedingungen, die insbesondere an den jeweiligen Mikroorganismus anzupassen sind, erforderlich sein. Den für die Fermentation optimalen pH-Wertbereich kann der Fachmann durch Routineexperimente ermitteln.

Um eine weitgehende Umsetzung der über das Medium (1) in das Fermentationsmedium eingebrachten Dextrine zu erzielen, wird man das Fermentationsmedium üblicherweise für einen Zeitraum von z. B. 2 bis 72 Stunden oder gegebenenfalls länger, z. B. 2 bis 96 Stunden, insbesondere von 5 bis 48 Stunden bei der eingestellten Temperatur halten. Typischerweise werden die durch Hydrolyse aus den Dextrinen erhaltenen Monosaccharide, insbesondere Glucose, sehr rasch von den Mikroorganismen verstoffwechselt, so dass in der Regel keine größeren Konzentrationen an Monosacchariden bzw. Glucose nachweisbar sind.

Die Durchführung der Fermentation kann in der dem Fachmann bekannten üblichen Art und Weise erfolgen. Hierzu wird man in der Regel den jeweils gewünschten Mikroorganismus in dem nach dem hier beschriebenen Verfahren erhaltenen Flüssigmedium kultivieren.

Das Fermentationsverfahren kann sowohl diskontinuierlich (Batchfahrweise) als auch semikontinuierlich (Fed-Batch-Fahrweise, einschließlich Fedbatch mit Zwischenernten) betrieben werden, wobei die semikontinuierliche Fahrweise bevorzugt ist.

Beispielsweise kann man das nach dem erfindungsgemäßen Verfahren erhaltene Medium (1) gegebenenfalls zusammen mit einer konventionellen Zuckerquelle, d. h. verstoffwechselbare Mono-, Di- und/oder Oligosaccharide oder Medien, die verstoffwechselbare Mono-, Di- und/oder Oligosaccharide enthalten, gegebenenfalls nach Verdünnen mit Wasser und Zugabe üblicher Medienbestandteile wie Puffer, Nährsalze, Stickstoffquellen wie Ammoniumsulfat, Harnstoff etc., komplexe Nährmedienbestandteile, enthaltend Aminosäuren, wie Hefeextrakte, Peptone, CSL und dergleichen, mit dem gewünschten Mikroorganismus inokulieren, und diesen unter Fermentationsbedingungen vermehren, bis die Mikroorganismenkonzentration den für die Fermentation gewünschten stationären Zustand erreicht. Hierbei werden die in dem Fermentationsmedium enthaltenen Dextrine verstoffwechselt und das gewünschte Stoffwechselprodukt wird gebildet (sogenannte Batchfahrweise oder Batchphase).

Bei der Fed-Batch-Fahrweise wird im Anschluss an die Batchphase, z. B. wenn die Gesamtzuckerkonzentration unter einen bestimmten Wert abgesunken ist, das Medium (1) kontinuierlich oder diskontinuierlich zu dem Fermentationsmedium gegeben.

Eine typische Ausgestaltung des erfindungsgemäßen Verfahrens ist die Fed-Batch-Fahrweise, welche die folgenden Schritte umfasst:
b1) Kultivieren des zur Überproduktion der organischen Verbindung befähigten Mikroorganismus in einem wässrigen Fermentationsmedium (2); und
b2) Zugabe des Dextrin-haltigen Mediums (1), gegebenenfalls zusammen mit einer konventionellen Zuckerquelle, zu dem Fermentationsmedium (2), in welchem die im Medium (1) enthaltenen Dextrine durch die die organische Verbindung überproduzierenden Mikroorganismen, gegebenenfalls nach vorheriger Verzuckerung, verstoffwechselt werden.

In Schritt b1) kann man beispielsweise zunächst ein herkömmliches zuckerhaltiges Medium, in der Regel eine Glucoselösung, oder ein erfindungsgemäßes Flüssigmedium (1) oder eine Mischung von (1) mit einer konventionellen Zuckerquelle, durch Verdünnung mit einer wässrigen Flüssigkeit, insbesondere Wasser, auf eine geeignete Zuckerkonzentration einstellen und die zur Fermentation üblichen Medienbestandteile wie Puffer, Nährsalze, Stickstoffquellen wie Ammoniumsulfat, Harnstoff etc., komplexe Nährmedienbestandteile, enthaltend Aminosäuren, wie Hefeextrakte, Peptone, CSL und dergleichen zugeben. Hierbei wird man das Verhältnis von Zuckermenge zu Flüssigkeit in der Regel vorzugsweise so wählen, dass die Gesamtkonzentration an Monosacchariden im Fermentationsmedium (2) weniger als 6 Gew.-%, z. B. im Bereich von ≥ 0 bis 5 Gew.-%, gerechnet als Glucoseäquivalente und bezogen auf das Gesamtgewicht des Fermentationsmediums (2), beträgt. Das so angesetzte zuckerhaltige Batch-Medium wird mit dem gewünschten Mikroorganismus inokuliert und der Mikroorganismus im Batch-Medium (Fermentationsmedium (2) unter Fermentationsbedingungen vermehrt, bis die Konzentration an Mikroorganismen einen für die Fermentation gewünschten stationären Zustand erreicht. Hierbei wird der in dem Fermentationsmedium (2) vorgelegte Zucker verstoffwechselt und das gewünschte Stoffwechselprodukt gebildet.

In der anschließenden Fed-Batch-Phase wird der Fermentationsprozess durch Zugabe des Dextrin-haltigen Mediums (1) zu dem Fermentationsmedium (2) aufrechterhalten und das vom Mikroorganismus überproduzierte Stoffwechselprodukt reichert sich in der Fermentationsbrühe an, wobei die Anreicherung intrazellulär als auch extrazellulär sein kann. Hierbei liegt das Volumenverhältnis von zugeführtem Medium (1) zu dem vorgelegten und die Mikroorganismen enthaltenden Batch-Medium (Fermentationsmedium (2)) im Allgemeinen im Bereich von etwa 1:10 bis 10:1, z. B. im Bereich von 1:5 bis 5:1 und insbesondere im Bereich von 1:1 bis 5:1. Insbesondere über die Zugabegeschwindigkeit des Mediums (1) kann die Zuckerkonzentration im Fermentationsmedium (2) reguliert werden. In der Regel wird man die Zugabegeschwindigkeit so einstellen, dass Gesamtzucker-Konzentration, d. h. die Summe aus Oligosacchariden und Monosacchariden einen Wert von 30 Gew.-%, insbesondere 20 Gew.-% nicht überschreitet. Die Konzentration an Monosaccharid in der Fermentationsbrühe liegt vorzugsweise im Bereich von > 0 Gew.-% bis etwa 5 Gew.-% und beträgt insbesondere nicht mehr als 3 Gew.-%.

In einer bevorzugten Ausführungsform umfasst das Fermentationsmedium (2) in Schritt b1) (d. h. hier das Batch-Medium) im Wesentlichen das dextrinhaltige Medium (1), die zur Überproduktion der organischen Verbindung befähigten Mikroorganismen, Medienbestandteile wie Puffer, Nährsalze, Stickstoffquellen wie Ammoniumsulfat, Harnstoff etc., komplexe Nährmedienbestandteile, enthaltend Aminosäuren, wie Hefeextrakte, Peptone, CSL und dergleichen und gegebenenfalls Wasser zur Verdünnung. Hierzu wird man ein Dextrin-haltiges Medium (1) gegebenenfalls auf den gewünschten Dextrin-Gehalt verdünnen, z. B. im Bereich von 15 bis 30 Gew.-%, gerechnet als Glucoseäquivalente und bezogen auf das Gesamtgewicht des Dextrin-haltigen Mediums (1), und dieses direkt zum Ansetzen des Fermentationsmediums (2) (Batch-Medium) verwenden.

Der Dextrin-Gehalt des zur Aufrechterhaltung der Fermentation eingesetzten Dextrin-haltigen Mediums gemäß Schritt b2), liegt üblicherweise höher, z. B. in den o. g. Bereichen, um die Verdünnung des Fermentationsmediums (2) zu minimieren.

Vorzugsweise wird man so vorgehen, dass man ein Dextrin-haltiges Medium (1) mit einem höheren Gehalt an Dextrinen, z. B. mindestens 30 Gew.-%, speziell mindestens 40 Gew.-% und ganz speziell mindestens 50 Gew.-%, gerechnet als Glucoseäquivalente und bezogen auf das Gesamtgewicht des Dextrin-haltigen Mediums (1), herstellt. Dieses Medium (1) verwendet man dann einerseits gemäß Schritt b1) nach Verdünnung mit Wasser zum Ansetzen des Batch-Mediums (Fermentationsmedium (2)) und andererseits gemäß Schritt b2) zur Zugabe zu dem Fermentationsmedium (2).

Unter Verwendung des Dextrin-haltigen Mediums (1) können flüchtige und nichtflüchtige, insbesondere nichtflüchtige mikrobielle Stoffwechselprodukte mit mindestens 3 C-Atomen oder mit mindestens 2 C-Atomen und 1 N-Atom fermentativ hergestellt werden.

Hierbei werden unter nichtflüchtigen Produkten solche Verbindungen verstanden, die sich auf destillativem Weg nicht unzersetzt aus der Fermentationsbrühe gewinnen lassen. Diese Verbindungen weisen in der Regel einen Siedepunkt oberhalb des Siedepunktes von Wasser, häufig oberhalb 150 °C und insbesondere oberhalb 200 °C bei Normaldruck auf. In der Regel handelt es sich um Verbindungen, die bei Normalbedingungen (298 K, 101,3 kPa) in festem Zustand vorliegen.

Es ist jedoch auch möglich, das wässrige Dextrin-haltige Medium (1) in einer Fermentation zur Herstellung nichtflüchtiger mikrobieller Stoffwechselprodukte einzusetzen, die bei Normaldruck einen Schmelzpunkt unterhalb des Siedepunktes von Wasser oder/und eine ölige Konsistenz aufweisen.

Der Begriff nichtflüchtige mikrobielle Stoffwechselprodukte umfasst hierin insbesondere organische, gegebenenfalls 1 oder mehrere, z. B. 1, 2, 3 oder 4 Hydroxylgruppen tragende Mono-, Di- und Tricarbonsäuren mit vorzugsweise 3 bis 10 C-Atomen, z. B. Weinsäure, Itaconsäure, Bernsteinsäure, Propionsäure, Milchsäure, 3-Hydroxypropionsäure, Fumarsäure, Maleinsäure, 2,5-Furandicarbonsäure, Glutarsäure, Lävulinsäure, Gluconsäure, Aconitsäure und Diaminopimelinsäure, Zitronensäure; proteinogene und nicht-proteinogene Aminosäuren, z. B..Lysin, Glutamat, Methionin, Phenylalanin, Asparaginsäure, Tryptophan und Threonin; Purin- und Pyrimidinbasen; Nukleoside und Nukleotide, z. B. Nikotinamidadenindinukleotid (NAD) und Adenosin-5'-monophosphat (AMP); Lipide; gesättigte und ungesättigte Fettsäuren mit vorzugsweise 10 bis 22 C-Atomen, z. B. γ-Linolensäure, Dihomo-γ-Linolensäure, Arachidonsäure, Eicosapentaensäure und Docosahexaensäure; Diole mit vorzugsweise 3 bis 8 C-Atomen, z. B. Propandiol und Butandiol; mehrwertige (auch als höherwertige bezeichnet) Alkohole mit 3 oder mehr, z. B. 3, 4, 5 oder 6 OH-Gruppen, z. B. Glycerin, Sorbitol, Manitol, Xylitol und Arabinitol; langkettige (auch als längerkettige bezeichnet) Alkohole mit wenigstens 4 C-Atomen, z. B. mit 4 bis 22 C-Atomen, z. B. Butanol; Kohlenhydrate, z. B. Hyaluronsäure und Trehalose; aromatische Verbindungen, z. B. aromatische Amine, Vanillin und Indigo; Vitamine und Provitamine, z. B. Ascorbinsäure, Vitamin B₆, Vitamin B₁₂ und Riboflavin, Cofaktoren und so genannte Nutrazeutika; Proteine, z. B. Enzyme wie Amylasen, Pektinasen, saure, hybride oder neutrale Zellulasen, Esterasen wie Lipasen, Pankreasen, Proteasen, Xylanasen und Oxidoreduktasen wie Laccase, Katalase und Peroxidase, Glucanasen, Phytasen; Carotenoide, z. B. Lycopin, ß-Carotin, Astaxanthin, Zeaxanthin und Canthaxanthin; Ketone mit vorzugsweise 3 bis 10 C-Atomen und gegebenenfalls 1 oder mehreren Hydroxylgruppen, z. B. Aceton und Acetoin; Lactone, z. B. γ-Butyrolacton, Cyclodextrine, Biopolymere, z. B. Polyhydroxyacetat, Polyester, z. B. Polylactid, Polysaccharide, Polyisoprenoide, Polyamide; sowie Vorstufen und Derivate der vorgenannten Verbindungen. Weitere als nichtflüchtige mikrobielle Stoffwechselprodukte in Frage kommende Verbindungen sind von Gutcho in Chemicals by Fermentation, Noyes Data Corporation (1973), ISBN: 0818805086 beschrieben.

Der Begriff "Cofaktor" umfasst nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Beispiele solcher Moleküle sind NAD und Nikotinamidadenindinukleotidphosphat (NADP); die Vorstufe dieser Cofaktoren ist Niacin.

Der Begriff "Nutrazeutikum" umfasst Nahrungsmittelzusätze, die bei Pflanzen und Tieren, insbesondere dem Menschen, gesundheitsfördernd sind. Beispiele solcher Moleküle sind Vitamine, Antioxidantien und bestimmte Lipide, z. B. mehrfach ungesättigte Fettsäuren.

Insbesondere sind die hergestellten Stoffwechselprodukte unter Enzymen, Aminosäuren, Vitaminen, Disacchariden, aliphatischen Mono- und Dicarbonsäuren mit 3 bis 10 C-Atomen, aliphatischen Hydroxycarbonsäuren mit 3 bis 10 C-Atomen, Ketonen mit 3 bis 10 C-Atomen, Alkanolen mit 4 bis 10 C-Atomen und Alkandiolen mit 3 bis 10 und insbesondere 3 bis 8 C-Atomen ausgewählt.

Es versteht sich für den Fachmann, dass die derart auf fermentativem Weg hergestellten Verbindungen jeweils in der von den eingesetzten Mikroorganismen produzierten Enantiomerenform erhalten werden (sofern unterschiedliche Enantiomere existieren). So erhält man z. B. von den Aminosäuren in der Regel das jeweilige L-Enantiomer.

Die in der Fermentation eingesetzten Mikroorganismen richten sich in an sich bekannter Weise nach den jeweiligen mikrobiellen Stoffwechselprodukten, wie unten im Einzelnen erläutert. Sie können natürlichen Ursprungs oder genetisch modifiziert sein. Beispiele für geeignete Mikroorganismen und Fermentationsverfahren sind z. B. in Tabelle A angegeben.

**Tabelle A:**

| **Stoff** | **Mikroorganismus** | **Referenz** |
|---|---|---|
| Weinsäure | *Lactobacilli, (z.* B. *Lactobacillus delbrueckii)* | Rehm, H.-J.: Biotechnology, Weinheim, VCH, 1980 und 1993-1995; Gutcho, Chemicals by Fermentation, Noyes Data Corporation (1973), |
| Itaconsäure | *Aspergillus terreus, Aspergillus itaconicus* and | Jakubowska, in Smith u. Pateman (Hrsg.), Genetics Physiology of Aspergillus, London: Academic Press 1977; Miall, in Rose (Hrsg.), Economic Microbiology, Vol. 2, S. 47 -119, London: Academic Press 1978; US 3044941 (1962). |
| Bernsteinsäure | *Actinobacillus sp. 130Z, Anaerobiospirillum succiniproducens, Actinobacillus succinogenes, E. coli* | Int. J. Syst. Bacteriol. 26, 498 -504 (1976); EP 249773 (1987), Erf.: Lemme u. Datta; US 5504004 (1996), Erf.: Guettler, Jain u. Soni; Arch. Microbiol. 167, 332 -342 (1997); Guettler MV, Rumler D, Jain MK.,Actinobacillus succinogenes sp. nov., a novel succinic-acid-producing strain from the bovine rumen. Int J Syst Bacteriol. 1999 Jan;49 Pt 1:207-16; US5723322, US5573931, US5521075,WO99/06532,US5869301, US 5770435 |
| Hydroxypropionsäure | *Lactobacillus delbrückii, L. leichmannii* od. *Sporolactobacillus inulinus* | RÖMPP Online Version 2.2 |
| Propionsäure | *Propionibacterium, z. B. P*. *arabinosum, P*. *schermanii, P. freudenreichii, Clostridium propionicum,* | Rehm, H.-J.: Biotechnology, Weinheim, VCH, 1980 und 1993-1995; Gutcho, Chemicals by Fermentation, Noyes Data Corporation (1973), |
| Diaminopimelinsäure | *Corynebacterium glutamicum* | Rehm, H.-J.: Biotechnology, Weinheim, VCH, 1980 und 1993-1995; Gutcho, Chemicals by Fermentation, Noyes Data Corporation (1973), |
| Zitronensäure | *Aspergillus niger, Aspergillus wentii* | Crit. Rev. Biotechnol. 3, 331 -373 (1986); Food Biotechnol. 7, 221-234 (1993); 10, 13-27 (1996). |
| Aconitsäure | *Aspergillus niger, Aspergillus wentii* | Crit. Rev. Biotechnol. 3, 331 -373 (1986); Food Biotechnol. 7, 221-234 (1993); 10, 13-27 (1996).; Rehm, H.-J.: Biotechnology, Weinheim, VCH, 1980 und 1993-1995; |
| Äpfelsäure | *Aspergilli, z. B. Aspergillus flavus, A. niger, A. oryzae, Coryne-bacterium* | US 3063910 |
| Gluconsäure | *Aspergilli,* z. B. *A*. *niger* | Gutcho, Chemicals by Fermentation, Noyes Data Corporation (1973), |
| Buttersäure | *Clostridium (z. B. Clostridium acetobut-lyicum, C. butyricum)* | Rehm, H.-J.: Biotechnology, Weinheim, VCH, 1980 und 1993-1995; |
| Milchsäure | *Lactobacillus z. B. L. delbrückii, L. leichmannii,* | Rehm, H.-J.: Biotechnology, Weinheim, VCH, 1980 und 1993-1995; |
| Lysin | *Corynebacterium glutamicum* | Ikeda, M.: Amino Acid Production Process (2003), Adv. Biochem. Engin/Biotechnol 79, 1-35. |
| Glutamat | *Corynebacterium glutamicum* | Ikeda, M.: Amino Acid Production Process (2003), Adv. Biochem. Engin/Biotechnol 79, 1-35. |
| Methionin | *Corynebacterium glutamicum* | Ikeda, M.: Amino Acid Production Process (2003), Adv. Biochem. Engin/Biotechnol 79, 1-35. |
| Phenylalanin | *Corynebacterium glutamicum, E.coli* | Trends Biotechnol. **3**, 64 -68 (1985); J. Ferment. Bioeng. 70, 253-260 (1990). |
| Threonin | *E. coli* | Ikeda, M.: Amino Acid Production Process (2003), Adv. Biochem. Engin/Biotechnol 79, 1-35. |
| Asparaginsäure | *E. coli* | Ikeda, M.: Amino Acid Production Process (2003), Adv. Biochem. Engin/Biotechnol 79, 1-35+dort. zit. Lit., Gutcho, Chemicals by Fermentation, Noyes Data Corporation (1973) |
| Purin- und Pyrimidinbasen | *Bacillus subtilis* | Rehm, H.-J.: Biotechnology, Weinheim, VCH, 1980 und 1993-1995; Gutcho, Chemicals by Fermentation, Noyes Data Corporation (1973), |
| Nikotinamidadenindinukleotid (NAD) | *Bacillus subtilis* | Rehm, H.-J.: Biotechnology, Weinheim, VCH, 1980 und 1993-1995; Gutcho, Chemicals by Fermentation, Noyes Data Corporation (1973), |
| Adenosin-5'-monophosphat (AMP) | *Bacillus subtilis* | Rehm, H.-J.: Biotechnology, Weinheim, VCH, 1980 und 1993-1995; Gutcho, Chemicals by Fermentation, Noyes Data Corporation (1973), |
| γ-Linolensäure | *Mucor, Mortiella, Aspergillus* spp. | Gill, I., Rao, V.: Polyunsaturated fatty acids, part 1: occurence, biological activities and applications (1997). Trends in Biotechnology 15 (10), 401-409; Zhu, H.: Utilization of Rice Brain by Pythium irregulare for Lipid Production. Master Thesis Lousiana State University, 31.10.2002 (URN etd-1111102-205855). |
| Dihomo-γ-Linolensäure | *Mortiella, Conidiobolus, Saprolegnia* spp. | Gill, I., Rao, V.: Polyunsaturated fatty acids, part 1: occurence, biological activities and applications (1997). Trends in Biotechnology 15 (10), 401-409; Zhu, H.: Utilization of Rice Brain by Pythium irregulare for Lipid Production. Master Thesis Lousiana State University, 31.10.2002 (URN etd-1111102-205855). |
| Arachidonsäure | *Mortiella, Phytium* spp. | Gill, I., Rao, V.: Polyunsaturated fatty acids, part 1: occurence, biological activities and applications (1997). Trends in Biotechnology 15 (10), 401-409; Zhu, H.: Utilization of Rice Brain by Pythium irregulare for Lipid Production. Master Thesis Lousiana State University, 31.10.2002 (URN etd-1111102-205855). |
| Eicosapentaensäure | *Mortiella, Phytium* spp., *Rhodopseudomonas, Shewanella* spp. | Gill, I., Rao, V.: Polyunsaturated fatty acids, part 1: occurence, biological activities and applications (1997). Trends in Biotechnology 15 (10), 401-409; Zhu, H.: Utilization of Rice Brain by Pythium irregulare for Lipid Production. Master Thesis Lousiana State University, 31.10.2002 (URN etd-1111102-205855). |
| Docosahexaensäure | *Thraustochytrium, Entomophthora* spp., *Rhodopseudomonas, Shewanella* spp. | Gill, I., Rao, V.: Polyunsaturated fatty acids, part 1: occurence, biological activities and applications (1997). Trends in Biotechnology 15 (10), 401-409; Zhu, H.: Utilization of Rice Brain by Pythium irregulare for Lipid Production. Master Thesis Lousiana State University, 31.10.2002 (URN etd-1111102-205855). |
| Propandiol | *E. coli* | DE 3924423, US 440379, WO 9635799, US 5164309 |
| Butandiol | *Enterobacter aerogenes" Bacillus subtilis, Klebsiella oxytoca* | Rehm, H.-J.: Biotechnology, Weinheim, VCH, 1980 und 1993-1995; Gutcho, Chemicals by Fermentation, Noyes Data Corporation (1973); H. G. SCHLEGEL and H. W. JANNASCH, 1981; Afschar et al.: Mikrobielle Produktion von 2,3-Butandiol. CIT 64 (6), 2004, 570-571 |
| Butanol | *Clostridium (z. B*. *Clostridium acetobutylicum, C. propionicum)* | Rehm, H.-J.: Biotechnology, Weinheim, VCH, 1980 und 1993-1995; Gutcho, Chemicals by Fermentation, Noyes Data Corporation (1973), |
| Glycerin | *Hefe, Saccharomyces rouxii* | Gutcho, Chemicals by Fermentation, Noyes Data Corporation (1973), |
| Mannitol | *Aspergillus candida, Torulopsis mannitofaciens* | Gutcho, Chemicals by Fermentation, Noyes Data Corporation (1973), |
| Arabitol | *Saccharomyces rouxii, S. mellis, Sclerotium glucanicum, Pichia ohmeri* | Gutcho, Chemicals by Fermentation, Noyes Data Corporation (1973), |
| Xylitol | *Saccharomyces cerevisiae* | Gutcho, Chemicals by Fermentation, Noyes Data Corporation (1973), |
| Hyaluronsäure | Streptococcus sp. | Rehm, H.-J.: Biotechnology, Weinheim, VCH, 1980 und 1993-1995; |
| Trehalose | *Brevibacterium, Corynebacterium, Microbacterium, Arthrobacter* spp., *Pleurotus genus , Filobasidium floriforme* | JP 05099974, JP 06311891, FR 2671099, EP 0555540, JP 3053791, Miyazaki, J.-I., Miyagawa, K.-I., Sugiyama, Y.: Trehalose Accumulation by Basidiomy-cotinous Yeast, Filobasidium floriforme. Journal of Fermentation and Bioengineering 81, (1996) 4, 315-319. |
| Ascorbinsäure | *Gluconobacter melanogenes* | RÖMPP Online Version 2.2 |
| Vitamin B₁₂ | *Propionibacterium* spp., *Pseudomonas denitrificans* | Chem. Ber. 1994, 923 -927; RÖMPP Online Version 2.2 |
| Riboflavin | *Bacillus subtilis, Ashbya gossypii* | WO 01/011052, DE 19840709, WO 98/29539, EP 1186664; Fujioka, K.: New biotechnology for riboflavin (vitamin B₂) and character of this ri-boflavin. Fragrance Journal (2003), 31 (3), 44-48. |
| Vitamin B₆ | *Rhizobium tropici, R*. *meliloti* | EP0765939 |
| Enzyme | *Apergilli (z. B. Aspergillusniger A.* oryzae), *Trichoderma , E.coli, Hansenula oder Pichia (z*. *B. Pichia pa- storius), Bacillus (z. B. Bacillus licheniformis, B. subtilis) u. v. a*. | Rehm, H.-J.: Biotechnology, Weinheim, VCH, 1980 und 1993-1995; Gutcho, Chemicals by Fermentation, Noyes Data Corporation (1973), |
| Zeaxanthin | *Dunaliella salina* | Jin et al (2003) Biotech.Bioeng. 81:115-124 |
| Canthaxanthin | *Brevibacterium* | Nelis et al (1991) J Appl Bacteriol 70:181-191 |
| Lycopin | *Blakeslea trispora, Candida utilis* | WO 03/056028, EP 01/201762, WO 01/12832, WO 00/77234, Miura et al (1998) Appl Environ Microbiol 64:1226-1229 |
| ß-Carotin | *Blakeslea trispora, Candida utilis* | Kim S., Seo W., Park Y., Enhanced production of beta-carotene from Blakeslea trispora with Span 20, Biotechnology Letters, Vol 19, No 6, 1997, 561-562; Mantouridou F., Roukas T.: Effect of the aeration rate and agitation speed on beta-carotene production and morphology of Blakeslea trispora in a stirred tank reactor: mathematical modelling, Biochemical Engineering Journal 10 (2002), 123-135; WO 93/20183; WO 98/03480, Miura et al (1998) Appl Environ Microbiol 64:1226-1229 |
| Astaxanthin | *Phaffia rhodozyma; Candida utilis* | US 5,599,711; WO 91/02060, Miura et al (1998) Appl Environ Microbiol 64:1226-1229 |
| Polyhydroxyalkanoate, Polyester | *Escherchia coli, Alcaligenes latus, u.v.a.* | S. Y. Lee, Plastic Bacteria, Progress and prospects for polyhydroxyalkanoate production in bacteria, Tibtech, Bd. 14, (1996), S. 431-438., Steinbüchel, 2003; Steinbüchel (Hg.), Biopolymers, 1. Aufl., 2003, Wiley-VCH, Weinheim und dort zitierte Literatur |
| Polysaccharide | *Leuconostoc mesenteroides, L. dextranicum, Xanthomonas campestris, u. v. a.* | Rehm, H.-J.: Biotechnology, Weinheim, VCH, 1980 und 1993-1995; Gutcho, Chemicals by Fermentation, Noyes Data Corporation (1973), |
| Polyisoprenoide | *Lactarius sp., Hygrophorus sp., Russula* sp. | Steinbüchel (Hg.), Biopolymers, 1. Aufl., 2003, Wiley-VCH,Weinheim und dort zitierte Literatur |
| Aceton | *Clostridium (z.* B. *Clostridium acetobutylicum, C. propionicum)* | Rehm, H.-J.: Biotechnology, Weinheim, VCH, 1980 und 1993-1995; Gutcho, Chemicals by Fermentation, Noyes Data Corporation (1973) |
| Acetoin | *Enterobacter aerogenes, Clostridium acetobutylicum, Lactococcus lactis* | Lengeler, J.W., Drews, G., Schlegel, H.G.: Hrsg., Biology of the Procaryotes, Thieme, Stuttgart (1999), S.307; RÖMPP Online-Edition |
| Vanillin | *Pseudomonas putida, Amycolatopsis sp.* | Priefert, H., Rabenhorst, J., Seinbüchel, A. Biotechnological production of vanillin. Appl. Microbiol. Biotechnol. 56, 296-314 (2001) |
| Thurigensin | *Bacillus thuringiensis* | Jian-Zhong Jong et al.: Fed-batch culture of Bacillus thuringiensis for thuringensin production in a tower type bioreactor. Biotechnology and Bioengineering 48 (3) (2004), 207-213. |
| Polyketide | *Streptomyces fradiae, Sorangium cellulosum* | Kirst: Fermentation-derived compounds as a source for new products. Pure & Appl. Chem. 70 (2), (1998), 335-338; Zirkle et al.: Heterologous production of the antifungal polyketide antibiotic soraphen A of Sorangium cellulosum So ce26 in Streptomyces lividans. Microbiology 150 (8), (2004), 2761-74. |
| Gibberellinsäure | *Gibberella fujikuroi* | Hollmann et al.: Extraktiv-Fermentation von Gibberellinsäure mit Gibberella fujikuroi. CIT 7 (1995), 892-895. |
| Indigo | *Escherichia coli JB 102* | Berry, A., Dodge, T.C., Pepsin, M., Weyler, W.: Application of metabolic engineering to improve both the production and use of biotech indigo. Journal of Industrial Microbiology & Biotechnology 28 (2002), 127-133. |

In bevorzugten Ausführungsformen der Erfindung ist die hergestellte organische Verbindung unter gegebenenfalls Hydroxylgruppen tragenden Mono-, Di- und Tricarbonsäuren mit 3 bis 10 C-Atomen, proteinogenen und nicht-proteinogenen Aminosäuren, Purinbasen, Pyrimidinbasen; Nukleosiden, Nukleotiden, Lipiden; gesättigten und ungesättigten Fettsäuren; Diolen mit 4 bis 10 C-Atomen, mehrwertigen Alkoholen mit 3 oder mehr Hydroxylgruppen, langkettigen Alkoholen mit wenigstens 4 C-Atomen, Kohlenhydraten, aromatischen Verbindungen, Vitaminen, Provitaminen, Cofaktoren, Nutrazeutika, Proteinen, Carotenoiden, Ketonen mit 3 bis 10 C-Atomen, Lactonen, Biopolymeren und Cyclodextrinen ausgewählt.

Eine erste bevorzugte Ausführungsform der Erfindung betrifft den Einsatz des erfindungsgemäß erhältlichen zuckerhaltigen Flüssigmediums in einer fermentativen Herstellung von Enzymen wie Phytasen, Xylanasen oder Glucanasen.

Eine zweite bevorzugte Ausführungsform der Erfindung betrifft den Einsatz des erfindungsgemäß erhältlichen zuckerhaltigen Flüssigmediums in einer fermentativen Herstellung von Aminosäuren wie Lysin, Methionin, Threonin und Glutamat

Eine weitere bevorzugte Ausführungsform der Erfindung betrifft den Einsatz des erfindungsgemäß erhältlichen zuckerhaltigen Flüssigmediums in einer fermentativen Herstellung von Vitaminen wie Pantothensäure und Riboflavin, Vorläufern und Folgeprodukten davon.

Eine besonders bevorzugte Ausführungsform der Erfindung betrifft die fermentative Herstellung von
- Mono-, Di- und Tricarbonsäuren, insbesondere aliphatischen Mono-, Di-, und Tricarbonsäuren mit 3 bis 10 C-Atomen wie Propionsäure, Fumarsäure, Bernsteinsäure, Itaconsäure, Citronensäure und Dimethylmalonsäure,
- aliphatischen Hydroxycarbonsäuren mit 3 bis 10 C-Atomen wie Milchsäure und 3-Hydroxypropionsäure;
- langkettigen Alkanolen wie zuvor genannt, insbesondere Alkanolen mit 4 bis 10 C-Atomen wie Butanol;
- Diolen wie zuvor genannt, insbesondere Alkandiolen mit 3 bis 10 und insbesondere 3 bis 8 C-Atomen wie Propandiol;
- Ketonen wie zuvor genannt, insbesondere Ketonen mit 3 bis 10 C-Atomen wie Aceton; und
- Kohlehydraten wie zuvor genannt, insbesondere Disacchariden wie Trehalose.

In einer weiteren besonders bevorzugten Ausführungsform handelt es sich bei dem in der Fermentation von den Mikroorganismen produzierten Stoffwechselprodukt um Polyhydroxyalkanoate wie Poly-3-hydroxybutyrat und Copolyester mit anderen organischen Hydroxycarbonsäuren wie 3-Hydroxyvaleriansäure, 4-Hydroxybuttersäure und anderen, die in Steinbüchel (a.a.O.) beschrieben sind, z. B. auch langkettige (auch als längerkettige bezeichnet) Hydroxycarbonsäuren wie 3-Hydroxyoctansäure, 3-Hydroxydecansäure und 3-Hydroxytetradecansäure, sowie Mischungen davon. Zur Durchführung der Fermentation können hier analoge Bedingungen und Vorgehensweisen angewendet werden, wie sie für andere Kohlenstoffquellen beschrieben wurden, z. B. in S. Y. Lee, Plastic Bacteria Progress and prospects for polyhydroxyalkanoate production in bacteria, Tibtech, Bd. 14, (1996), S. 431-438.

In einer bevorzugten Ausführungsform sind die in der Fermentation eingesetzten Mikroorganismen daher ausgewählt unter natürlichen oder rekombinanten Mikroorganismen, die wenigstens eines der folgenden Stoffwechselprodukte überproduzieren:
- Enzyme wie Phytase, Xylanase oder Glucanase, insbesondere Phytase;
- Aminosäuren wie Lysin, Threonin oder Methionin, insbesondere Lysin und Methionin; ,
- Vitamine wie Pantothensäure und Riboflavin; Vorläufer und/oder Folgeprodukte davon;
- Disaccharide wie Trehalose;
- aliphatische Mono-, Di- und Tricarbonsäuren mit 3 bis 10 C-Atomen wie Propionsäure, Fumarsäure, Bernsteinsäure, Itaconsäure, Citronensäure und Dimethylmalonsäure;
- Polyhydroxyalkanoate wie Poly-3-hydroxybutyrat und Copolyester der 3-Hydroxybuttersäure;
- aliphatische Hydroxycarbonsäuren mit 3 bis 10 C-Atomen wie Milchsäure und 3-Hydroxypropionsäure;
- Ketone mit 3 bis 10 C-Atomen wie Aceton;
- Alkanole mit 4 bis 10 C-Atomen wie Butanol; und
- Alkandiole mit 3 bis 8 C-Atomen wie Propandiol.

Geeignete Mikroorganismen sind üblicherweise ausgewählt unter den Gattungen Corynebacterium, Bacillus, Ashbya, Escherichia, Aspergillus, Alcaligenes, Actinobacillus, Anaerobiospirillum, Lactobacillus, Propionibacterium, Rhizopus und Clostridium, insbesondere unter Stämmen von Corynebacterium glutamicum, Bacillus subtilis, Ashbya gossypii, Escherichia coli, Aspergillus niger oder Alcaligenes latus, Anaerobiospirillum succiniproducens, Actinobacillus succinogenes, Lactobacillus delbrückii, Lactobacillus leichmannii, Propionibacterium arabinosum, Propionibacterium schermanii, Propionibacterium freudenreichii, Clostridium propionicum, Clostridium formicoaceticum, Clostridium acetobutylicum, Rhizopus arrhizus und Rhizopus oryzae.

In einer bevorzugten Ausführungsform handelt es sich bei dem in der Fermentation eingesetzten Mikroorganismus um einen Stamm aus der Gattung Corynebacterium, insbesondere um einen Stamm des Corynebacterium glutamicum. Insbesondere handelt es sich um einen Stamm der Gattung Corynebacterium, speziell des Corynebacterium glutamicum, welcher eine Aminosäure, speziell Lysin, Methionin oder Glutamat überproduziert.

In einer weiteren bevorzugten Ausführungsform handelt es sich bei dem in der Fermentation eingesetzten Mikroorganismus um einen Stamm aus der Gattung Escherichia, insbesondere um einen Stamm des Escherichia coli. Insbesondere handelt es sich um einen Stamm der Gattung Escherichia, speziell des Escherichia coli, welcher eine Aminosäure, speziell Lysin, Methionin oder Threonin überproduziert.

In einer speziellen bevorzugten Ausführungsform handelt es sich bei dem in der Fermentation von den Mikroorganismen produzierten Stoffwechselprodukt um Lysin. Zur Durchführung der Fermentation können hier analoge Bedingungen und Vorgehensweisen angewendet werden, wie sie für andere Kohlenstoffquellen beschrieben wurden, z. B. in Pfefferle et al., a.a.O., und US 3,708,395. Prinzipiell kommen sowohl eine kontinuierliche als auch eine diskontinuierliche (Batch oder Fed-Batch) Betriebsweise in Betracht, bevorzugt ist die Fed-Batch Betriebsweise.

In einer weiteren besonders bevorzugten Ausführungsform handelt es sich bei dem in der Fermentation von den Mikroorganismen produzierten Stoffwechselprodukt um Methionin. Zur Durchführung der Fermentation können hier analoge Bedingungen und Vorgehensweisen angewendet werden, wie sie für andere Kohlenstoffquellen beschrieben wurden, z. B. in WO 03/087386 und WO 03/100072.

In einer weiteren besonders bevorzugten Ausführungsform handelt es sich bei dem in der Fermentation von den Mikroorganismen produzierten Stoffwechselprodukt um Pantothensäure. Zur Durchführung der Fermentation können hier analoge Bedingungen und Vorgehensweisen angewendet werden, wie sie für andere Kohlenstoffquellen beschrieben wurden, z. B. in der WO 01/021772.

In einer weiteren besonders bevorzugten Ausführungsform handelt es sich bei dem in der Fermentation von den Mikroorganismen produzierten Stoffwechselprodukt um Riboflavin. Zur Durchführung der Fermentation können hier analoge Bedingungen und Vorgehensweisen angewendet werden, wie sie für andere Kohlenstoffquellen beschrieben wurden, z. B. in WO 01/011052, DE 19840709, WO 98/29539, EP 1186664 und Fujioka, K.: New biotechnology for riboflavin (vitamin B2) and character of this riboflavin. Fragrance Journal (2003), 31 (3), 44-48.

In einer weiteren besonders bevorzugten Ausführungsform handelt es sich bei dem in der Fermentation von den Mikroorganismen produzierten Stoffwechselprodukt um Fumarsäure. Zur Durchführung der Fermentation können hier analoge Bedingungen und Vorgehensweisen angewendet werden, wie sie für andere Kohlenstoffquellen beschrieben wurden, z. B. in Rhodes et al, Production of Fumaric Acid in 20-L Fermentors, Applied Microbiology, 1962, 10 (1), 9-15.

In einer weiteren besonders bevorzugten Ausführungsform handelt es sich bei dem in der Fermentation von den Mikroorganismen produzierten Stoffwechselprodukt um Bernsteinsäure. Zur Durchführung der Fermentation können hier analoge Bedingungen und Vorgehensweisen angewendet werden, wie sie für andere Kohlenstoffquellen beschrieben wurden, z. B. in Int. J. Syst. Bacteriol. 26, 498 -504 (1976); EP 249773 (1987), Erf.: Lemme u. Datta; US 5504004 (1996), Erf.: Guettler, Jain u. Soni; Arch. Microbiol. 167, 332 -342 (1997); Guettler MV, Rumler D, Jain MK.,Actinobacillus succinogenes sp. nov., a novel succinic-acid-producing strain from the bovine rumen. Int J Syst Bacteriol. 1999 Jan; 49 Pt 1:207-16; US 5,723,322, US 5,5739,31, US 5,521,075, WO99/06532, US 5,869,301 oder US 5,770,435.

In einer weiteren besonders bevorzugten Ausführungsform handelt es sich bei dem in der Fermentation von den Mikroorganismen produzierten Stoffwechselprodukt um eine Phytase. Zur Durchführung der Fermentation können hier analoge Bedingungen und Vorgehensweisen angewendet werden, wie sie für andere Kohlenstoffquellen beschrieben wurden, z. B. in der WO 98/55599.

Bei der Fermentation resultiert eine Fermentationsbrühe, die neben dem gewünschten mikrobiellen Stoffwechselprodukt im Wesentlichen die während der Fermentation erzeugte Biomasse, die nicht verstoffwechselten Bestandteile der verflüssigten Stärkelösung und insbesondere die nicht-stärkehaltigen festen Bestandteile der Stärkequelle, wie z. B. Fasern und nicht verwertete Zucker, sowie nicht verwertete Puffer- und Nährsalze enthält. Dieses Flüssigmedium wird in der vorliegenden Anmeldung auch als Fermentationsbrühe bezeichnet, wobei die Fermentationsbrühe auch das zugegebene Dextrin-haltige Medium (1) umfasst, bei dem eine erst teilweise oder unvollständige fermentative Umsetzung der darin enthaltenen Zucker, d. h. eine teilweise oder unvollständige mikrobielle Verstoffwechselung der verwertbaren Zucker (z. B. Mono- und Disaccharide), erfolgt ist.

Vor der Isolierung oder Abreicherung eines mikrobiellen Stoffwechselprodukts oder Abtrennung der flüchtigen Bestandteile der Fermentationsbrühe, wird gegebenenfalls ein Sterilisierungsschritt in der oben beschriebenen Weise durchgeführt.

Eine spezielle Ausführungsform (I) der Erfindung betrifft ein Verfahren, bei dem man wenigstens ein mikrobielles Stoffwechselprodukt aus der Fermentationsbrühe abreichert oder isoliert. Die flüchtigen Bestandteile der Fermentationsbrühe werden anschließend weitgehend entfernt, wobei man eine feste oder halbfeste Proteinzusammensetzung erhält. Eine genauere Beschreibung zur Durchführung eines solchen Verfahrens und der dabei erhaltenen Proteinzusammensetzung ist Gegenstand der WO 2005/116228 (PCT/EP2005/005728) der Anmelderin, auf die bezüglich weiterer Details Bezug genommen wird.

Die Isolierung oder Abreicherung des Stoffwechselprodukts aus der Fermentationsbrühe erfolgt in der Regel derart, dass man wenigstens ein Stoffwechselprodukt so aus der Fermentationsbrühe abreichert oder isoliert, dass der Gehalt dieses Stoffwechselprodukts in der verbleibenden Fermentationsbrühe höchstens 20 Gew.-%, insbesondere höchstens 10 Gew.-%, speziell höchstens 5 Gew.-% und ganz speziell höchstens 2,5 Gew.-%, jeweils bezogen auf das Gesamtgewicht der verbleibenden Fermentationsbrühe, beträgt.

Die Isolierung oder Abreicherung von Feinchemikalien (d. h. des mikrobiellen Stoffwechselprodukts) aus der Fermentationsbrühe kann in einem oder mehreren Schritte erfolgen. Ein wesentlicher Schritt dabei ist die Abtrennung der festen Bestandteile aus der Fermentationsbrühe. Diese kann entweder vor oder nach der Isolierung des Wertprodukts durchgeführt werden. Sowohl zur Isolierung von Wertstoffen als auch zur Abtrennung von Feststoffen, d. h. Fest-Flüssig-Phasenseparation, sind im Fachgebiet übliche Methoden bekannt, die auch Schritte zur Grob- und Feinreinigung der Wertstoffe sowie zur Konfektionierung umfassen (z. B. beschrieben in Belter, P. A, Bioseparations: Downstream Processing for Biotechnology, John Wiley & Sons (1988), und Ullmann's Encyclopedia of Industrial Chemistry, 5. Aufl. auf CD-ROM, Wiley-VCH).

Zur Isolierung des Wertprodukts kann man vorteilhafterweise so vorgehen, dass man zunächst die festen Bestandteile aus der Fermentationsbrühe entfernt, z. B. mittels Zentrifugation oder Filtration, und anschließend das Wertprodukt aus der flüssigen Phase isoliert, z. B. durch Kristallisation, Fällung, Adsorption oder Destillation. Alternativ kann das Wertprodukt auch direkt aus der Fermentationsbrühe isoliert werden, z. B. durch Einsatz chromatographischer Verfahren oder von Extraktions-Verfahren. Als chromatographisches Verfahren ist insbesondere die lonenaustauschchromatographie zu nennen, bei der das Wertprodukt selektiv auf der Chromatographiesäule isoliert werden kann. In diesem Fall erfolgt die Abtrennung der Feststoffe aus der verbleibenden Fermentationsbrühe vorteilhafterweise z. B. durch Dekantieren, Eindampfen oder/und Trocknung.

Im Falle flüchtiger oder öliger Verbindungen ist in der Regel eine Kontrolle der maximalen Temperaturen während der Aufarbeitung, insbesondere während der Trocknung erforderlich. Vorteilhaft können diese Verbindungen auch dadurch hergestellt werden, dass man sie in quasi fester Form (pseudofester Form) auf Adsorbentien formuliert. Zu diesem Zweck geeignete Adsorbentien sind z. B. in der WO 2005/116228 (PCT/EP2005/005728) der Anmelderin angegeben. Beispiele für Verbindungen, die vorteilhaft auf diese Weise hergestellt werden können, sind γ-Linolensäure, Dihomo-γ-Linolensäure, Arachidonsäure, Eicosapentaensäure und Docosahexaensäure, weiterhin Propionsäure, Milchsäure, Propandiol, Butanol und Aceton. Auch diese Verbindungen in pseudofester Formulierung werden im Sinne der vorliegenden Erfindung als nichtflüchtige mikrobielle Stoffwechselprodukte in fester Form verstanden.

Eine weitere spezielle Ausführungsform (II) betrifft ein Verfahren, bei dem man die flüchtigen Bestandteile der Fermentationsbrühe ohne vorherige Isolierung oder Abreicherung eines nichtflüchtigen mikrobiellen Stoffwechselprodukts, und gegebenenfalls ohne vorherige Abtrennung fester Bestandteile, weitgehend entfernt, wobei man eine feste Formulierung eines nichtflüchtigen mikrobiellen Stoffwechselprodukts erhält. Eine genauere Beschreibung zur Durchführung eines solchen Verfahrens findet sich in der PCT/EP2006/066057 (die ältere Patentanmeldung DE 10 2005 042 541.0) der Anmelderin.

Weitgehend bedeutet, dass nach Entfernung der flüchtigen Bestandteile ein fester oder zumindest halbfester Rückstand verbleibt, der sich gegebenenfalls durch Zusatz von Feststoffen in ein festes Produkt überführen lässt. In der Regel bedeutet dies, die flüchtigen Bestandteile bis zu einem Restfeuchtegehalt von nicht mehr als 30 Gew.-%, häufig nicht mehr als 20 Gew.-% und insbesondere nicht mehr als 15 Gew.-%, zu entfernen. In der Regel wird man die flüchtigen Bestandteile der Fermentationsbrühe vorteilhafterweise bis auf einen Restfeuchtigkeitsgehalt im Bereich von 0,2 bis 30 Gew.-%, bevorzugt 1 bis 20 Gew.-%, besonders bevorzugt 2 bis 15 Gew.-% und ganz besonders bevorzugt 5 bis 15 Gew.-%, bezogen auf das nach Trocknung ermittelte Gesamtgewicht der festen Bestandteile, aus der Fermentationsbrühe entfernen. Der Restfeuchtigkeitsgehalt kann durch übliche dem Fachmann bekannte Verfahren bestimmt werden, z. B. mittels Thermogravimetrie (Hemminger et al., Methoden der thermischen Analyse, Springer Verlag, Berlin, Heidelberg, 1989).

Die Gewinnung des(der) nichtflüchtigen Stoffwechselprodukts(-produkte) in fester Form aus der Fermentationsbrühe kann in ein, zwei oder mehreren Stufen erfolgen, insbesondere in einem ein- oder zweistufigen Vorgehen. In der Regel wird mindestens eine, insbesondere die abschließende Stufe zur Gewinnung des Stoffwechselprodukts in fester Form einen Trocknungsschritt umfassen.

Bei der einstufigen Vorgehensweise wird man die flüchtigen Bestandteile der Fermentationsbrühe, gegebenenfalls nach vorgenannter Vorabtrennung, entfernen, bis der gewünschte Restfeuchtigkeitsgehalt erreicht ist.

Bei der zwei- oder mehrstufigen Vorgehensweise wird man die Fermentationsbrühe, zunächst aufkonzentrieren, z. B. mittels (Mikro-, Ultra-)Filtration oder thermisch durch Verdampfen eines Teils der flüchtigen Bestandteile. Der Anteil der flüchtigen Bestandteile, die in dieser Stufe entfernt werden beträgt in der Regel 10 bis 80 Gew.-% und insbesondere 20 bis 70 Gew.-%, bezogen auf das Gesamtgewicht der flüchtigen Bestandteile der Fermentationsbrühe. In einer oder mehreren sich anschließenden Stufen werden die restlichen flüchtigen Bestandteile der Fermentationsbrühe entfernt, bis der gewünschte Restfeuchtigkeitsgehalt erreicht ist.

Das Entfernen der flüchtigen Bestandteile des Flüssigmediums erfolgt gemäß dieser Ausführungsform (II) im Wesentlichen ohne eine vorherige Abreicherung oder gar Isolierung des Wertprodukts. Folglich wird bei der Entfernung der flüchtigen Bestandteile der Fermentationsbrühe das nichtflüchtige Stoffwechselprodukt im Wesentlichen nicht zusammen mit den flüchtigen Bestandteilen des Flüssigmediums entfernt, sondern verbleibt mit wenigstens einem Teil, üblicherweise mit der Hauptmenge und insbesondere mit der Gesamtmenge der sonstigen festen Bestandteile aus der Fermentationsbrühe im so erhaltenen Rückstand. Dementsprechend können aber auch - vorzugsweise geringe - Anteile des gewünschten nichtflüchtigen mikrobiellen Stoffwechselprodukts, in der Regel maximal 20 Gew.-%, z. B. 0,1 bis 20 Gew:-%, bevorzugt nicht mehr als 10, insbesondere nicht mehr als 5 Gew.-%, besonders bevorzugt maximal 2,5 Gew.-% und ganz besonders bevorzugt maximal 1 Gew.-%, bezogen auf das Gesamttrockengewicht des Stoffwechselprodukts, beim Entfernen der flüchtigen Bestandteile der Fermentationsbrühe zusammen mit diesen entfernt werden. In einer ganz besonders bevorzugten Ausführungsform verbleibt das gewünschte nichtflüchtige mikrobielle Stoffwechselprodukt zu wenigstens 90 Gew.-%, insbesondere wenigstens 95 Gew.-%, speziell 99 Gew.-% und ganz speziell etwa 100 Gew.-%, jeweils bezogen auf das Gesamttrockengewicht des Stoffwechselprodukts, als Feststoff in Mischung mit dem nach Entfernen der flüchtigen Bestandteile erhaltenen Teil oder der Gesamtheit der festen Bestandteile des Fermentationsmediums.

Sofern gewünscht, kann vor dem Entfernen der flüchtigen Bestandteile ein Teil, z. B. 5 bis 80 Gew.-% und insbesondere 30 bis 70 Gew.-%, der nicht-stärkehaltigen festen Bestandteile aus der Fermentationsbrühe abgetrennt werden, z. B. mittels Zentrifugation oder Filtration. Gegebenenfalls wird man eine solche Vorabtrennung durchführen, um gröbere Feststoffpartikel, die keine oder nur geringe Anteile an nichtflüchtigem mikrobiellem Stoffwechselprodukt enthalten, zu entfernen. Zur Vorfiltration können dem Fachmann bekannte, übliche Verfahren, z. B. unter Verwendung grobmaschiger Siebe, Netze, Lochbleche, oder dergleichen angewendet werden. Gegebenenfalls kann eine Abtrennung grober Feststoffpartikel auch in einem Fliehkraftabscheider erfolgen. Die hierbei eingesetzten Apparaturen wie Dekanter, Zentrifugen, Sedikanter und Separatoren sind dem Fachmann ebenfalls bekannt .Auf diese Weise erhält man einen festen oder halbfesten, z. B. pastösen Rückstand, welcher das nichtflüchtige Stoffwechselprodukt und die nichtflüchtigen, in der Regel festen nicht-stärkehaltigen Bestandteile der Stärkequelle oder zumindest große Teile davon, häufig wenigstens 90 Gew.-% oder die Gesamtmenge der festen nicht-strärkehaltigen Bestandteile enthält.

Durch Zugabe von Formulierungshilfsmitteln wie Träger- und Coatingmaterialien, Bindemitteln sowie anderer Additive können die Eigenschaften des getrockneten und zusammen mit den festen Bestandteilen der Fermentation vorliegenden Stoffwechselprodukts gezielt hinsichtlich verschiedener Parameter wie Wirkstoffgehalt, Korngröße, Partikelform, Neigung zum Stauben, Hygroskopizität, Stabilität, insbesondere Lagerstabilität, Farbe, Geruch, Fließverhalten, Agglomerationsneigung, elektrostatischer Aufladung, Licht- und Temperaturempfindlichkeit, mechanischer Stabilität und Redispergierbarkeit in an sich bekannter Weise konfektioniert werden.

Zu den üblicherweise verwendeten Formulierungshilfsmitteln gehören z. B. Bindemittel, Trägermaterialien, Puderungs-/Fließhilfsmittel, ferner Farbpigmente, Biozide, Dispergiermittel, Antischaummittel, viskositätsregulierende Mittel, Säuren, Laugen, Antioxidantien, Enzymstabilisatoren, Enzyminhibitoren, Adsorbate, Fette, Fettsäuren, Öle oder Mischungen hieraus. Derartige Formulierungshilfsmittel werden vorteilhaft insbesondere bei Anwendung von Formulierungs- und Trocknungsverfahren wie Sprüh-, Wirbelschicht- und Gefriertrocknung als Trocknungshilfsmittel eingesetzt. Wegen weiterer Details wird auf die PCT/EP2006/066057 (ältere Anmeldung DE 10 2005 042 541.0) verwiesen.

Der Anteil an den vorgenannten Zusatzstoffen und gegebenenfalls weiteren Zusatzstoffen wie Coatingmaterialien kann je nach den speziellen Erfordernissen des jeweiligen Stoffwechselprodukts sowie in Abhängigkeit von den Eigenschaften der eingesetzten Zusatzstoffe stark variieren und z. B. im Bereich von 0,1 bis 80 Gew.-% und insbesondere im Bereich von 1 bis 30 Gew.%, jeweils bezogen auf das Gesamtgewicht des fertig formulierten Produkts bzw. Stoffgemischs, liegen.

Die Zugabe von Formulierungshilfsmitteln kann vor, während oder nach der Aufarbeitung der Fermentationsbrühe (auch als Produktformulierung oder Feststoffdesign bezeichnet) und insbesondere während der Trocknung erfolgen. Eine Zugabe von Formulierungshilfsmitteln vor Aufarbeitung der Fermentationsbrühe bzw. des Stoffwechselprodukts kann insbesondere vorteilhaft sein, um die Prozessierbarkeit der aufzuarbeitenden Stoffe bzw. Produkte zu verbessern. Die Formulierungshilfsmittel können sowohl dem in fester Form erhaltenen Stoffwechselprodukt als auch einer dieses enthaltenden Lösung oder Suspension zugegeben werden, z. B. nach abgeschlossener Fermentation direkt zu der Fermentationsbrühe oder zu einer im Verlauf der Aufarbeitung erhaltenen Lösung oder Suspension vor dem abschließenden Trocknungsschritt.

So können die Hilfsstoffe z. B. in eine Suspension des mikrobiellen Stoffwechselprodukts eingemischt werden; eine solche Suspension kann auch auf ein Trägermaterial gegeben werden, z. B. durch Aufsprühen oder Untermischen. Die Zugabe von Formulierungshilfsstoffen während der Trocknung kann z. B. eine Rolle spielen, wenn eine das Stoffwechselprodukt enthaltende Lösung oder Suspension versprüht wird. Insbesondere nach der Trocknung erfolgt eine Zugabe von Formulierungshilfsmitteln z. B. beim Aufbringen von Überzügen bzw. Coatings/Coatingschichten auf getrocknete Partikel. Sowohl nach dem Trocknen als auch nach einem eventuellen Coatingschritt können dem Produkt weitere Hilfsmittel zugegeben werden.

Das Entfernen der flüchtigen Bestandteile aus der Fermentationsbrühe erfolgt in an sich bekannter Weise durch übliche Verfahren zur Abtrennung fester Phasen von flüssigen Phasen, einschließlich Filtrationsverfahren und Verfahren zum Verdampfen flüchtiger Bestandteile der flüssigen Phasen. Derartige Verfahren, die auch Schritte zur Grobreinigung der Wertstoffe sowie Schritte zur Konfektionierung umfassen können, werden z. B. in Belter, P. A, Bioseparations: Downstream Processing for Biotechnology, John Wiley & Sons (1988), und Ullmann's Encyclopedia of Industrial Chemistry, 5. Aufl. auf CD-ROM, Wiley-VCH, beschrieben. Im Rahmen der Produktformulierung bzw. der Aufarbeitung nach abgeschlossener Fermentation anwendbare, dem Fachmann bekannte Verfahren, Apparaturen, Hilfsstoffe bzw. allgemeine und spezielle Ausführungsformen sind ferner in EP 1038 527, EP 0648 076, EP 835613, EP 0219 276, EP 0394 022, EP 0547 422, EP 1088 486, WO 98/55599, EP 0758 018 und WO 92/12645 beschrieben.

In einer ersten Variante dieser Ausführungsform (II) wird man das nichtflüchtige mikrobielle Stoffwechselprodukt, sofern es in der flüssigen Phase gelöst vorliegt, aus der flüssigen Phase in die feste Phase überführen, z. B. durch Kristallisation oder Fällung. Anschließend erfolgt eine Abtrennung der nichtflüchtigen festen Bestandteile einschließlich des Stoffwechselprodukts, z. B. mittels Zentrifugation, Dekantieren oder Filtration. In ähnlicher Weise kann man auch ölige Stoffwechselprodukte abtrennen, wobei man die jeweiligen öligen Fermentationsprodukte durch Zusatz von Adsorbentien, z. B. Kieselsäure, Kieselgele, Lehm, Ton und Aktivkohle, in eine feste Form überführt.

In einer zweiten Variante dieser Ausführungsform (II) werden die flüchtigen Bestandteile durch Verdampfen entfernt. Das Verdampfen kann in an sich bekannter Weise erfolgen. Beispiele für geeignete Verfahren zum Verdampfen flüchtiger Bestandteile sind die Sprühtrocknung, Wirbelschichttrocknung bzw. -agglomeration, Gefriertrocknung, Strom- und Kontakttrocknern sowie Extrusionstrocknung. Auch eine Kombination der vorgenannten Verfahren mit formgebende Verfahren wie Extrusion, Pelletierung oder Prilling kann vorgenommen werden. Bei diesen letztgenannten Verfahren werden vorzugsweise teilweise oder weitgehend vorgetrocknete Stoffwechselprodukt-haltige Stoffgemische eingesetzt.

In einer bevorzugten Ausführungsform umfasst das Entfernen der flüchtigen Bestandteile der Fermentationsbrühe ein Verfahren zur Sprühtrocknung oder ein Verfahren der Wirbelschichttrocknung, einschließlich der Wirbelschichtgranulierung. Hierzu wird die Fermentationsbrühe, gegebenenfalls nach einer Vorabtrennung zur Entfernung grober Feststoffpartikel, die keine oder nur geringe Anteile an nichtflüchtigem mikrobiellen Stoffwechselprodukt enthalten, einer oder mehreren Sprüh- oder Wirbelschichttrocknungsapparaturen zugeführt. Der Transport bzw. die Zuführung der feststoffbeladenen Fermentationsbrühe erfolgt zweckmäßigerweise mittels üblicher Transportvorrichtungen für feststoffhaltige Flüssigkeiten, z. B. Pumpen wie Exzenterschneckenpumpen (z. B. der Fa. Delasco PCM) oder Hochdruckpumpen (z. B. der Fa. LEWA Herbert Ott GmbH).

Die Durchführung einer Fermentation unter Verwendung des erfindungsgemäßen zuckerhaltigen Flüssigmediums kann auch derart erfolgen, dass man
(i) dem in Schritt a2) erhaltenen Dextrin-haltigen Medium (1), das nichtstärkehaltige feste Bestandteile der Stärkequelle enthält, eine Teilmenge von nicht mehr als 50 Gew.-%, z. B. im Bereich von 5 bis 45 Gew.-%, bezogen auf das Gesamtgewicht, entnimmt und die Restmenge einer Fermentation zur Her- . stellung eines ersten Stoffwechselprodukts (A), z. B. eines nichtflüchtigen Stoffwechselprodukts (A) in fester Form oder eines flüchtigen Stoffwechselprodukts (A), zuführt; und
(ii) diese Teilmenge, gegebenenfalls nach vorheriger vollständiger oder teilweiser Abtrennung der nicht-stärkehaltigen festen Bestandteile der Stärkequelle, einer Fermentation zur Herstellung eines zweiten Stoffwechselprodukts (B), das mit dem Stoffwechselprodukt (A) identisch oder davon verschieden ist, zuführt.

Im Falle der Abtrennung der nicht-stärkehaltigen festen Bestandteile gemäß (ii) beträgt der Feststoffgehalt des verbleibenden Anteils des zuckerhaltigen Flüssigmediums bevorzugt maximal 50 Gew.-%, insbesondere maximal 30 Gew.-%, besonders bevorzugt maximal 10 Gew.-% und ganz besonders bevorzugt maximal 5 Gew.-% beträgt. Insbesondere ist es in diesem Fall bevorzugt, die gesamten Feststoffe vor der Fermentation zur Herstellung des zweiten Stoffwechselprodukts (B) abzutrennen.

Diese Vorgehensweise ermöglicht es, in der separaten Fermentation gemäß (ii) Mikroorganismen einzusetzen, für die bestimmte Mindestanforderungen erfüllt sein müssen, z. B. bezüglich der Sauerstofftransferrate. Für solche in der separaten Fermentation gemäß (ii) eingesetzten Mikroorganismen kommen z. B. *Bacillus species,* bevorzugt *Bacillus subtilis* in Betracht. Die durch derartige Mikroorganismen in der separaten Fermentation produzierten Verbindungen sind insbesondere unter Vitaminen, Cofaktoren und Nutrazeutika, Purin- und Pyrimidinbasen, Nukleosiden und Nukleotiden, Lipiden, gesättigten und ungesättigten Fettsäuren, aromatischen Verbindungen, Proteinen, Carotenoiden, speziell unter Vitaminen, Cofaktoren und Nutrazeutika, Proteinen und Carotenoiden und ganz speziell unter Riboflavin und Calciumpantothenat ausgewählt.

Eine bevorzugte Ausgestaltung dieser Vorgehensweise betrifft die parallel betriebene Herstellung gleicher Stoffwechselprodukte (A) und (B) in zwei separaten Fermentationen. Dies ist insbesondere dann vorteilhaft, wenn für verschiedene Anwendungen des gleichen Stoffwechselprodukts unterschiedliche Anforderungen an die Reinheit zu stellen sind. Demnach wird das erste Stoffwechselprodukt (A), z. B. eine als Futtermitteladditiv zu verwendende Aminosäure, z. B. Lysin, Methionin, Threonin oder Glutamat, unter Einsatz der feststoffhaltigen Fermentationsbrühe und das gleiche zweite Stoffwechselprodukt (B), z. B. die gleiche als Nahrungsmitteladditiv zu verwendende Aminosäure unter Einsatz der gemäß ii) feststoffabgereicherten Fermentationsbrühe hergestellt. Durch die vollständige oder teilweise Abtrennung der nicht-stärkehaltigen festen Bestandteile kann der Reinigungsaufwand bei der Aufarbeitung des Stoffwechselprodukts, dessen Anwendungsbereich eine höhere Reinheit erfordert, z. B. als Nahrungsmitteladditiv, reduziert werden.

In einer weiteren bevorzugten Ausführungsform dieser Vorgehensweise kann z. B. wie folgt vorgegangen werden. Man implementiert eine vorzugsweise großvolumige Fermentation zur Herstellung von Stoffwechselprodukten A, z. B. von Aminosäuren wie Lysin, Methionin, Glutamat oder Threonin, von Citronensäure oder von Ethanol, z. B. entsprechend den in der WO 2005/116228 (PCT/EP2005/005728) oder PCT/EP2006/066057 (die ältere Patentanmeldung DE 10 2005 042 541.0) beschriebenen Verfahren oder entsprechend den zur fermentativen Herstellung von Bioethanol bekannten Verfahren. Gemäß i) wird ein Teil des nach Schritt a2) erhaltenen Mediums (1) entnommen. Der gemäß i) entnommene Teil kann gemäß ii) durch übliche Verfahren, z. B. Zentrifugation oder Filtration, vollständig oder teilweise von den Feststoffen befreit werden, je nach den Erfordernissen der Fermentation zur Herstellung von B. Das so gewonnene, gegebenenfalls vollständig oder teilweise von den Feststoffen befreite Medium (1) wird gemäß ii) einer Fermentation zur Produktion eines Stoffwechselprodukts B zugeführt. Ein gemäß i) abgetrennter Feststoffstrom wird vorteilhafterweise zum Strom des Mediums (1) der großvolumigen Fermentation zurück gegeben.

Wenn das in der großvolumigen Fermentation hergestellte mikrobielle Stoffwechselprodukt (A) Ethanol ist, so weist das nach dem Schritt ii) hergestellte Medium (1) Konzentrationen an Oligosacchariden auf, wie sie bei der fermentativen Ethanol-Produktion (Bioethanol) üblich sind, z. B. im Bereich von 20 bis 33 Gew.-%. Die Durchführung einer Abtrennung von Feststoffen gemäß Schritt ii) richtet sich auch hier nach den Erfordernissen der Fermentation zur Herstellung des jeweiligen Stoffwechselprodukts B.

In einer bevorzugten Ausführungsform der vorstehend beschriebenen Vorgehensweise handelt es sich bei dem in der Fermentation von den Mikroorganismen produzierten Stoffwechselprodukt B um Riboflavin. Zur Durchführung der Fermentation können hier analoge Bedingungen und Vorgehensweisen angewendet werden, wie sie für andere Kohlenstoffquellen beschrieben wurden, z. B. in der WO 01/011052, DE 19840709, WO 98/29539, EP 1186664 und Fujioka, K.: New biotechnology for riboflavin (vitamin B2) and character of this riboflavin. Fragrance Journal (2003), 31(3), 44-48.

Zur Durchführung dieser Variante des Verfahrens implementiert man eine vorzugsweise großvolumige Fermentation zur Herstellung von Stoffwechselprodukten A, z. B. von Aminosäuren wie Lysin, Methionin, Glutamat oder, von Citronensäure oder von Ethanol, wie oben beschrieben. Gemäß i) wird ein Teil des nach Schritt a2) erhaltenen Mediums (1) entnommen und gemäß ii) durch übliche Verfahren, z. B. Zentrifugation oder Filtration, vollständig oder teilweise von den Feststoffen befreit. Das daraus gewonnene, im Wesentlichen vollständig oder teilweise von den Feststoffen befreite Medium (1) wird gemäß ii) einer Fermentation zur Produktion des Stoffwechselprodukts B, hier Riboflavin, zugeführt. Der gemäß ii) abgetrennte Feststoffstrom wird vorteilhafterweise zum Strom des Mediums (1) der großvolumigen Fermentation zurück gegeben.

Die auf diese Art und Weise erzeugte, Riboflavin-haltige Fermentationsbrühe kann nach analogen Bedingungen und Vorgehensweisen aufgearbeitet werden, wie sie für andere Kohlenstoffquellen beschrieben wurden, z. B. in DE 4037441, EP 464582, EP 438767 und DE 3819745. Nach Lyse der Zellmasse erfolgt die Abtrennung des kristallin vorliegenden Riboflavins vorzugsweise durch Dekantieren. Andere Arten der Feststoffabtrennung, z. B. Filtration, sind ebenfalls möglich. Anschließend wird das Riboflavin getrocknet, bevorzugt mittels Sprüh- und Wirbelschichttrocknern. Alternativ dazu kann das gemäß ii) erzeugte, Riboflavin-haltige Fermentationsgemisch nach analogen Bedingungen und Vorgehensweisen aufgearbeitet werden, wie z. B. in der EP 1048668 und EP 730034 beschrieben. Nach Pasteurisierung wird die Fermentationsbrühe hier zentrifugiert und die zurückbleibende feststoffhaltige Fraktion mit einer mineralischen Säure behandelt. Das gebildete Riboflavin wird aus dem wässrig-sauren Medium filtriert, gegebenenfalls gewaschen und anschließend getrocknet.

In einer weiteren bevorzugten Ausführungsform dieser Vorgehensweise handelt es sich bei dem in der Fermentation von den Mikroorganismen produzierten Stoffwechselprodukt B um Pantothensäure. Zur Durchführung der Fermentation können hier analoge Bedingungen und Vorgehensweisen angewendet werden, wie sie für andere Kohlenstoffquellen beschrieben wurden, z. B. in der WO 01/021772.

Zur Durchführung dieser Variante des Verfahrens kann z. B. wie oben für Riboflavin beschrieben vorgegangen werden. Das gemäß i) vorgereinigte, vorzugsweise im Wesentlichen von den Feststoffen befreite, Medium (1) wird einer Fermentation gemäß ii) zur Produktion von Pantothensäure zugeführt. Dabei ist die im Vergleich zum feststoffhaltigen Flüssigmedium verringerte Viskosität besonders vorteilhaft. Der abgetrennte Feststoffstrom wird vorzugsweise zum Strom des zuckerhaltigen Flüssigmediums (1) der großvolumigen Fermentation zurückgegeben.

Die gemäß ii) hergestellte Pantothensäure-haltige Fermentationsbrühe kann nach analogen Bedingungen und Vorgehensweisen aufgearbeitet werden, wie sie für andere Kohlenstoffquellen beschrieben wurden, z. B. in der EP 1050219 und WO 01/83799. Nach Pasteurisieren der gesamten Fermentationsbrühe werden die verbleibenden Feststoffe z. B. durch Zentrifugation oder Filtration abgetrennt. Der Klärlauf der Feststoffabtrennung wird teilweise eingedampft, gegebenenfalls mit Calciumchlorid versetzt und getrocknet, insbesondere sprühgetrocknet.

Die abgetrennten Feststoffe können im Rahmen des parallel betriebenen, großvolumigen Fermentationsverfahrens zusammen mit dem jeweiligen gewünschten mikrobiellen Stoffwechselprodukt (A) gewonnen werden.

Nach der Trocknung und/oder Formulierung bzw. Konfektionierung können der Produktformulierung bzw. Proteinzusammensetzung ganze oder gemahlene Getreidekörner, bevorzugt Mais, Weizen, Gerste, Hirse, Triticale und/oder Roggen zugegeben werden.

Die nachfolgenden Beispiele dienen der Veranschaulichung einzelner Aspekte der vorliegenden Erfindung, sie sind jedoch in keiner Weise als einschränkend zu verstehen.

### Beispiele

### 1. Vermahlen der Stärkequelle

Die im Folgenden eingesetzten Mahlgüter wurden wie folgt hergestellt. Ganze Maiskörner wurden unter Verwendung einer Rotormühle vollständig vermahlen. Unter Einsatz unterschiedlicher Schlägerwerke, Mahlbahnen bzw. Siebeinbauten wurden dabei drei verschiedene Feinheiten erzielt. Eine Siebanalyse des Mahlgutes mittels Labor-Vibrationssieb (Vibrations-Analysenmaschine: Retsch Vibrotronic Typ VE1; Siebzeit 5 min; Amplitude: 1,5 mm) ergab die in Tabelle 1 aufgeführten Ergebnisse.

**Tabelle 1**

| **Versuchsnummer** | **T 70/03** | **T 71/03** | **T 72/03** |
|---|---|---|---|
| < 2 mm / % | 99,4 | 100 | 100 |
| < 0,8 mm / % | 66 | 100 | 99 |
| < 0,63 mm / % | 58,6 | 98,5 | 91 |
| < 0,315 mm / % | 48,8 | 89 | 65 |
| < 0,1 mm / % | | 25 | 9,6 |
| < 0,04 mm / % | | 8 | 3,2 |
| Mahlgutmenge gesamt | 20 kg | 11,45 kg | 13,75 kg |

### II. Enzymatische Stärkeverflüssigung und -verzuckerung

### 11.1. Ohne Phytase im Verzuckerungsschritt

### II.1a) Enzymatische Stärkeverflüssigung

Aus 320 g trocken vermahlenem Maismehl (T71/03) wurde unter stetem Rühren eine Suspension in 480 g Wasser angesetzt und mit 310 mg Calciumchlorid versetzt. Das Rühren wurde während der gesamten Versuchsdurchführung fortgesetzt. Nach Einstellung des pH-Wertes mit H₂SO₄ auf 6,5 und Erhitzen auf 35 °C wurden 2,4 g Termamyl 120L Typ L (Novozymes A/S) zugegeben. Über 40 min wurde das Reaktionsgemisch auf eine Temperatur von 86,5 °C erhitzt, wobei der pH gegebenenfalls mit NaOH auf den zuvor eingestellten Wert nachjustiert wurde. Innerhalb von 30 min wurden weitere 400 g des trocken vermahlenen Maismehls (T71/03) zugegeben, wobei die Temperatur auf 91 °C erhöht wurde. Das Reaktionsgemisch wurde ca. 100 min bei dieser Temperatur gehalten. Anschließend wurden weitere 2,4 g Termamyl 120L zugegeben und die Temperatur ca. 100 min gehalten. Der Fortschritt der Verflüssigung wurde während der Durchführungsdauer mit der lod-Stärke-Reaktion verfolgt. Die Temperatur wurde abschließend auf 100 °C erhöht und das Reaktionsgemisch weitere 20 min gekocht. Zu diesem Zeitpunkt war keine Stärke mehr nachzuweisen. Der Reaktor wurde auf 35 °C abgekühlt.

### II.3 Weitere Arbeitsvorschriften zur enzymatischen Stärkeverflüssigung

### II.3a) Maismehl

360 g deionisiertes Wasser wurden in einem Reaktionsgefäß vorgelegt. 1,54 ml CaCl₂ Stammlösung (100 g CaCl₂ x 2 H₂O / l) wurden bis zu einer Endkonzentration von etwa 70 ppm Ca²⁺ in der Maische zugegeben. Unter stetem Rühren ließ man 240 g Maismehl langsam in das Wasser einrieseln. Nach Einstellung des pH auf 6,5 mit 50 gew.-%iger wässriger NaOH-Lösung gab man 4,0 ml (= 2 Gew.-% Enzym/Trockenmasse) Termamyl 120 L Typ L (Novozymes A/S) zu. Die Maische wurde dann schnell auf bis zu 85°C erhitzt. Hierbei musste der pH fortwährend kontrolliert und gegebenenfalls angepasst werden.

Nach Erreichen der endgültigen Temperatur begann man die Zugabe von weiterem Mehl, zunächst von 50 g Mehl. Zusätzlich gab man der Maische 0,13 ml CaCl₂ Stammlösung zu, um die Ca²⁺-Konzentration bei 70 ppm zu halten. Während der Zugabe hielt man die Temperatur konstant bei 85 °C. Man wartete mindestens 10 min ab, um eine vollständige Reaktion zu gewährleisten, bevor man eine weitere Portion (50 g Mehl und 0,13 ml CaCl₂ Stammlösung) zugab. Nach Zugabe von zwei Portionen gab man 1,67 ml Termamyl zu; anschließend gab man zwei weitere Portionen (jeweils 50 g Mehl und 0,13 ml CaCl₂ Stammlösung) zu. Es wurde ein Gehalt an Trockenmasse von 55 Gew.-% erreicht. Nach der Zugabe erhöhte man die Temperatur auf 100 °C und kochte die Maische 10 min.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g lod und 10 g Kaliumiodid pro Liter) zu. Eine tiefblaue Färbung zeigte einen Gehalt an verbleibender Stärke an; eine Braunfärbung trat ein, wenn die Stärke vollständig hydrolysiert wurde. Wenn der Test einen Anteil verbleibender Stärke anzeigte, wurde die Temperatur erneut auf 85 °C erniedrigt und konstant gehalten. Man gab weitere 1,67 ml Termamyl zu, bis die lod-Stärke-Reaktion negativ ausfiel.

### II.3b) Roggenmehl (inklusive Cellulase/Hemicellulase-Vorbehandlung)

360 g deionisiertes Wasser wurden in einem Reaktionsgefäß vorgelegt. Unter stetem Rühren ließ man 155 g Roggenmehl langsam in das Wasser einrieseln. Die Temperatur wurde konstant bei 50 °C gehalten. Nach Einstellung des pH auf 5,5 mit 50 gew.-%iger wässriger NaOH-Lösung gab man 3,21 ml (= 2,5 Gew.-% Enzym/TrocKenmasse) Viscozyme L (Novozymes A/S) zu. Nach 30 min startete man die Zugabe von weiterem Mehl, zunächst gab man 55 g Mehl zu. Nach weiteren 30 min gab man erneut 50 g Mehl hinzu; 30 min später noch einmal 40 g Mehl. 30 min nach der letzten Zugabe konnte mit der Verflüssigung begonnen werden.

1,7 ml CaCl₂ Stammlösung (100 g CaCl₂ x 2 H₂O / l) wurden zugegeben. Nach Einstellung des pH auf 6,5 mit 50 gew.-%iger wässriger NaOH-Lösung gab man 5,0 ml (= 2 Gew.-% Enzym/Trockenmasse) Termamyl 120 L Typ L (Novozymes A/S) zu. Die Maische wurde dann schnell auf 85 °C erhitzt. Hierbei wurde der pH fortwährend kontrolliert und gegebenenfalls angepasst.

Nach Erreichen der endgültigen Temperatur begann man die Zugabe von weiterem Mehl, zunächst von 60 g Mehl. Zusätzlich gab man der Maische 0,13 ml CaCl₂ Stammlösung zu, um die Ca²⁺-Koncentration bei 70 ppm zu halten. Während der Zugabe hielt man die Temperatur konstant bei 85 °C. Man wartete mindestens 10 min ab, um eine vollständige Reaktion zu gewährleisten, bevor man eine, weitere Portion (40 g Mehl und 0,1 ml CaCl₂ Stammlösung) zugab. Man gab 1,1 ml Termamyl zu; anschließend gab man eine weitere Portionen (40 g Mehl und 0,1 ml CaCl₂ Stammlösung) zu. Es wurde ein Gehalt an Trockenmasse von 55 Gew.-% erreicht. Nach der Zugabe erhöhte man die Temperatur auf 100 °C und kochte die Maische 10 min.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g lod und 10 g Kaliumiodid pro Liter) zu. Eine tiefblaue Färbung zeigte einen Gehalt an verbleibender Stärke an; eine Braunfärbung trat ein, wenn die Stärke vollständig hydrolysiert wurde. Wenn der Test einen Anteil verbleibender Stärke anzeigte, wurde die Temperatur erneut auf 85 °C erniedrigt und konstant gehalten. Man gab weitere 1,1 ml Termamyl zu, bis die lod-Stärke-Reaktion negativ ausfiel.

### II.3c) Weizenmehl (inklusive Xylanase-Vorbehandlung)

360 g deionisiertes Wasser wurden in einem Reaktionsgefäß vorgelegt. Das Wasser wurde auf 55 °C erhitzt und der pH mit 50 gew.-%iger wässriger NaOH-Lösung auf 6,0 eingestellt. Nach Einstellung von Temperatur und pH gab man 3,21 ml (= 2,5 Gew.-% Enzym/Trockenmasse) Shearzyme 500L (Novozymes A/S) zu. Unter stetem Rühren ließ man 155 g Weizenmehl langsam in die Lösung einrieseln. Die Temperatur und der pH wurden konstant gehalten. Nach 30 min startete man die Zugabe von weiterem Mehl, zunächst gab man 55 g Mehl zu. Nach weiteren 30 min gab man 50 g Mehl hinzu; 30 min später noch einmal 40 g Mehl. 30 min nach der letzten Zugabe konnte mit der Verflüssigung begonnen werden.

Die Verflüssigung wurde wie unter II.3b beschrieben durchgeführt.
III. Stamm
ATCC13032 lysC^{fbr}

In einigen der nachfolgenden Beispiele wurde ein modifizierter Stamm von Corynebacterium glutamicum eingesetzt, der unter der Bezeichnung ATCC13032 lyscfbr in der WO 05/059144 beschrieben wurde.

### IV: Identifizierung Glucoamylase-exprimierender/produzierender Stämme

### IVa) Screening in Gendatenbanken

Eine Recherche auf Glucoamylase produzierende Stämme
1. Glycoamylase (1,4-alpha-D-glucan glucohydrolase) ist durch die folgende EC Nummer EC 3.2.1.3 klassifiziert [1].
2. Eine Recherche mit der Suchanfrage EC 3.2.1.3 wurde in den folgenden Datenbanken durchgeführt: Brenda, Swissprot, ERGO-WIT, CAZY und PIR, wobei man jeweils eine Liste von Proteinen erhielt, welche die EC 3.2.1.3 aufwiesen.
3. Die jeweiligen Ergebnislisten wurden vereinigt, und nach Treffern der taxonomischen Reiche Archaea, Bacteria und Fungi gefiltert und gemäß der Speziesnamen sortiert.
4. Die Species, welche das Filterkriterium des Absatzes 3 erfüllten und für die ein Glucoamylaseeintrag in wenigstens einer der in Absatz 2 genannten Datenbanken gefunden wurde, weisen mit hoher Wahrscheinlichkeit die Fähigkeit auf, Glucoamylase zu produzieren. Im Einzelnen handelt es sich hierbei um die folgenden Spezies:
   Agrobacterium tumefaciens, Arxula adeninivorans, Ashbya gossypii, Aspergillus awamori, Aspergillus candidus, Aspergillus foetidus, Aspergillus fumigatus, Aspergillus Kawachi, Aspergillus nidulans, Aspergillus niger, Aspergillus oryzae, Aspergillus phoenicis, Aspergillus saitoi, Aspergillus shirousami, Aspergillus terreus, Athelia rolfsii, Bacillus circulans, Bacillus stearothermophilus, Beta vulgaris, Bradyrhizobium japonicum, Burkholderia cenocepacia, Burkholderia fungorum, Burkholderia pseudomallei, Candida albicans, Candida antarctica, Candida glabrata, Candida tsukubaensis, Caulobacter crescentus, Cephalosporium charticola, Cephalosporium eichhorniae, Ceratocystis paradoxa, Chaetomium thermophilum, Chlorobium tepidum, Chromobacterium violaceum, Cladosporium resinae, Clostridium sp., Clostridium thermocellum, Clostridium thermosaccharolyticum, Coniophora puteana, Corticium rolfsii, Corynebacterium glutamicum, Cryptococcus neoformans, Debaryomyces hansenii, Debaryomyces occidentalis, Emericella nidulans, Endomyces sp., Endomycopsis fibuligera, Fusarium venenatum, Haloarcula marismortui, Hormoconis resinae, Humicola grisea, Humicola lanuginosa, Hypocrea lixii, Kluyveromyces lactis, Lentinula edodes, Lipomyces kononenkoae, Magnaporthe grisea, Mesorhizobium loti, Methanocaldococcus jannaschii, Methanococcus jannaschii, Methanococcus maripaludis, Methanosarcina acetivorans, Methanosarcina barkeri, Methanosarcina mazei, Monascus rubiginosus, Monascus sp., Mucor rouxianus, Mycobacterium bovis, Mycobacterium leprae, Mycobacterium marinum, Mycobacterium tuberculosis, Myrothecium sp., Neurospora crassa, Nostoc punctiforme, Oryza sativa, Paecilomyces variotii, Penaeus japonicus, Penicillium chrysogenum, Penicillium oxalicum, Picrophilus torridus, Pseudomonas fluorescens, Pseudomonas putida, Pseudomonas syringae, Ralstonia eutropha, Ralstonia metallidurans, Rana japonica, Rhizobium leguminosarum, Rhizopus delemar, Rhizopus javanicus, Rhizopus niveus, Rhizopus oryzae, Rhizopus sp., Rhodococcus sp., Rhodopseudomonas palustris, Rhodospirillum rubrum, Saccharomyces cerevisiae, Saccharomyces diastaticus, Saccharomycopsis fibuligera, Saccharomycopsis fibuligera, Schizosaccharomyces pombe, Schwanniomyces occidentalis, Shewanella oneidensis, Sphingomonas aromaticivorans, Streptomyces coelicolor, Sulfolobus acidocaldarius, Sulfolobus solfataricus, Talaromyces emersonii, Termitomyces clypeatus, Thermoactinomyces vulgaris, Thermoanaerobacter tengcongensis, Thermoanaerobacterium thermosaccharolyticum, Thermoascus crustaceus, Thermomyces lanuginosus, Thermoproteus tenax, Thielavia terrestris, Trichoderma reesei und Trichosporon adeninovorans.

### IVb) Screening im Schüttelkolbentest mit anschließendem Enzymaktivitätstest

Verschiedene Mikroorganismen werden in einem Schüttelkolbentest auf Glucoamylaseaktivität hin untersucht. Als Medium hierfür ist jedes konventionelle Medium nutzbar, das für das Wachstum des Organismus geeignet ist und zu einer Expression der Glucoamylase führt. Geeignete Medien sind im Handel erhältlich oder können entsprechend publizierter Vorschriften hergestellt werden (z. B. wie in Katalogen der American Type Culture Collection beschrieben).

Beispielsweise kann in einem definierten Medium als einzige Kohlenstoffquelle ein Gemisch von Glucoseoligomeren unterschiedlicher Kettenlänge eingesetzt werden. Da unter Fermentationsbedingungen keine thermische Hydrolyse der Oligosaccharide stattfindet, können nur Stämme mit einer Glucoamylase- und / oder Maltaseaktivität in diesem Medium wachsen. Als Substrat eignet sich z. B. Maldex 150 (Amylum Group). Das Screening wird unter verschiedenen Fermentationsbedingungen (pH, Temperatur) durchgeführt.

Um Glucoamylase- und Maltaseaktivität zu unterscheiden, wird vor dem Versuch die Oligosaccharidzusammensetzung der Mischung z. B. mittels HPLC analysiert. So hat z. B. Maldex 150 folgende Zusammensetzung (siehe Tabelle 2):

**Tabelle 2: Zusammensetzung von Maldex 150 (Amylum Group)**

| Polymerisationsgrad | [%] |
|---|---|
| DP1 | 1,1 |
| DP2 | 4,0 |
| DP3 | 7,4 |
| DP4 | 5,0 |
| DP5 | 4,8 |
| DP6 | 8,4 |
| DP7 | 9,6 |
| DP8 | 4,6 |
| DP9 | 3,5 |
| >DP9 | 51,6 |

Entsprechend dieser Analyse wird ein Kontrollmedium mit Maltose und Glucose angesetzt. Wachstum und Lysinproduktion im Oligosaccharidmedium, die über die Werte dieser Kontrolle hinausgehen, sind dann eindeutig einer Glucoamylaseaktivität zuzuschreiben.

Alternativ zu einem Maltodextringemisch können auch reine Maltotetraose, Maltopentaose usw. als Kohlenstoffquelle für ein Screening eingesetzt werden. Nach Abbruch der Kultivierung wird die Biomasse abzentrifugiert und der Überstand filtiriert.

Der klare Überstand wird in einem Glucoamylase-Aktivitätstest (CHEN et al., J. Gen. *Appl. Microbiol.,* 51, 175-181 (2005)) eingesetzt. Dazu wird eine Reaktionsmischung aus 0,2 ml 50mM Acetat-Natriumacetat-Puffer (pH 5,0) mit 0,5 % löslicher Stärke und 0,2 ml Überstand eingesetzt. Nach einer Reaktionszeit von 10 Minuten bei 60 °C wird die Reaktion durch 10 min Kochen bei 100 °C abgestoppt. Die freigesetzte Glucosemenge wird mit Hilfe der Glucoseoxidase/Peroxidase-Methode (Bergmayer und Bernt, 1974) bestimmt. Eine Unit Glucoamylaseaktivität ist dabei definiert als die Menge an Enzym, die unter den gegebenen Reaktionsbedingungen 1 µmol Glucose pro Minute aus löslicher Stärke freisetzt.

### IVc) Screening mit Hilfe von Primern/Sonden

Eine alternative Methode zur Überprüfung, ob der zu untersuchende Organismus glucoamylasecodierende Sequenzen besitzt, ist ein Screening mit Hilfe von Primern oder Sonden, die spezifisch für diese Sequenzen sind.
i) Ausgehend von konservierten Bereichen bekannter Glucoamylase-Gene wird eine Sonde konstruiert, um DNA-Sequenzen verschiedener Organismen zu iden-tifizieren und klonieren, die Polypeptide mit einer Glucoamylaseaktivität codieren. Insbesondere können solche Sonden für die Hybridisierung mit der genomischen oder cDNA des gewünschten Organismus genutzt werden, gefolgt von einem Southern Blot nach der Standardmethode, um das gesuchte Gen zu identifizieren.
   Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260).
ii) Ausgehend von konservierten Bereichen bekannter Glucoamylase-Gene werden PCR-Primer synthetisiert. Diese werden in einer PCR-Reaktion mit der DNA des zu untersuchenden Organismus eingesetzt. Sind entsprechende Bindestellen für die Primer, d. h. glucoamylasecodierende Gene, vorhanden, so können die entsprechenden amplifizierten Oligonukleotide mit Hilfe einer im Anschluss durchgeführten Gelelektrophorese identifiziert werden. Anleitungen zur Amplifikation von DNA-Sequenzen mit Hilfe der Polymerase-Kettenreaktion (PCR) findet der Fachmann unter anderem im Handbuch von Gait: Oligonucleotide sythesis: a practical approach (IRL Press, Oxford, UK, 1984) und bei Newton und Graham: PCR (Spektrum Akademischer Verlag, Heidelberg, Deutschland, 1994).

### Beispiel 1

Verflüssigtes Maismehlhydrolysat wurde in Schüttelkolbenversuchen unter Verwendung von *Corynebacterium glutamicum* eingesetzt.

### I) Verflüssigung

360 g deionisiertes Wasser wurden in einem Reaktionsgefäß vorgelegt. Unter stetem Rühren ließ man 240 g Maismehl langsam in das Wasser einrieseln. Nach Einstellung des pH auf 5,8 mit 50 gew.-%iger wässriger NaOH-Lösung gab man 4,0 ml (= 2 Gew.-% Enzym/Trockenmasse) Liquozyme SC (von Novozymes A/S) zu. Die Maische wurde dann schnell auf 85 °C erhitzt. Hierbei wurde der pH fortwährend kontrolliert und gegebenenfalls angepasst.

Nach Erreichen der endgültigen Temperatur begann man die Zugabe von weiterem Mehl, zunächst von 50 g Mehl. Während der Zugabe hielt man die Temperatur konstant bei 85 °C. Man wartete mindestens 10 min ab, um eine vollständige Reaktion zu gewährleisten, bevor man eine weitere Portion (50 g) Mehl zugab. Nach Zugabe von zwei Portionen gab man 1,67 ml Liquozyme zu; anschließend gab man zwei weitere Portionen (jeweils 50 g) Mehl zu. Es wurde ein Gehalt an Trockenmasse von 55 Gew.-% erreicht. Nach der Zugabe erhöhte man die Temperatur auf 100 °C und kochte die Maische 10 min.

Man entnahm eine Probe und kühlte diese auf Raumtemperatur. Nach Verdünnung der Probe mit deionisiertem Wasser (etwa 1:10) gab man einen Tropfen konzentrierte Lugolsche Lösung (Mischung von 5 g lod und 10 g Kaliumiodid pro Liter) zu. Eine tiefblaue Färbung zeigt einen Gehalt an verbleibender Stärke an; eine Braunfärbung tritt ein, wenn die Stärke vollständig hydrolysiert wurde. Die negativ auf Stärke getestete Mischung wurde heiß in sterile Behälter gefüllt und nach Abkühlung bei 4 °C gelagert.

### II) Fermentation mit Corynebacterium glutamicum

### Stamm

Es wurde der modifizierte Wildtyp mit feedback-deregulierter Aspartokinase ATCC13032 lyscfbr verwendet.

### Herstellung des Inokulums

Die Zellen wurden nach dem Ausstreichen auf sterilem CM+CaAc-Agar (Zusammensetzung: siehe Tabelle 3; 20 min bei 121 °C) über Nacht bei 30 °C inkubiert. Anschließend wurden die Zellen von den Platten abgekratzt und in Saline resuspendiert. 25 ml des Mediums (siehe Tabelle 4) in 250 ml Erlenmeyerkolben mit zwei Schikanen wurden jeweils mit einer solchen Menge der so hergestellten Zellsuspension angeimpft, dass die optische Dichte einen Wert von OD610 = 0,5 bei 610 nm erreichte.

**Tabelle 3: Zusammensetzung der CM+CaAc-Agarplatten**

| Konzentration | Inhaltsstoff |
|---|---|
| 10,0 g/l | D-Glucose |
| 2,5 g/l | NaCl |
| 2,0 g/l | Harnstoff |
| 5,0 g/l | Bacto Pepton (Difco) |
| 5,0 g/l | Hefeextrakt (Difco) |
| 5,0 g/l | Beef Extract (Difco) |
| 20,0 g/l | Casaminoacids |
| 20,0 g/l | Agar |

### Herstellung der Fermentationsbrühe

Die Zusammensetzungen des Kolbenmediums sind in Tabelle 4 aufgeführt. Der Versuch wurde in Dreifachbestimmung durchgeführt.

**Tabelle 4: Kolbenmedien**

| Maismehlhydrolysat | 180 g/l |
|---|---|
| (NH₄)SO₄ | 20 g/l |
| Harnstoff | 5 g/l |
| KH₂PO₄ | 0,113 g/l |
| K₂HPO₄ | 0,138 g/l |
| ACES | 52 g/l |
| MOPS | 21 g/l |
| Citronensäure x H₂O | 0,49 g/l |
| 3,4-Dihydroxybenzoesäure | 3,08 mg/l |
| NaCl | 2,5 g/l |
| KCI | 1 g/l |
| MgSO₄ x 7 H₂O | 0,3 g/l |
| FeSO₄ x 7 H₂O | 25 mg/l |
| MnSO₄ x 4 - 6H₂O | 5 mg/l |
| ZnCl₂ | 10 mg/l |
| CaCl₂ | 20 mg/l |
| H₃BO₃ | 150 µg/l |
| CoCl₂ x 6 H₂O | 100 µg/l |
| CuCl₂ x 2 H₂O | 100 µg/l |
| NiSO₄ x 6H₂O | 100 µg/l |
| Na₂MoO₄ x 2H₂O | 25 µg/l |
| Biotin (Vit. H) | 1050 µg/l |
| Thiamin x HCl (Vit B₁) | 2100 µg/l |
| Nicotinamid | 2,5 mg/l |
| Pantothensäure | 125 mg/l |
| Cyanocobalamin (Vit B₁₂) | 1 µg/l |
| 4-Aminobenzoesäure (PABA; Vit. H₁) | 600 µg/l |
| Folsäure | 1,1 µg/l |
| Pyridoxin (Vit. B₆) | 30 µg/l |
| Riboflavin (Vit. B₂) | 90 µg/l |
| CSL | 40 ml/l |
| pH* | 6,85 |

| | |
|---|---|
| * mit verdünnter wässriger NaOH-Lösung eingestellt | |

Nach dem Animpfen wurden die Kolben drei Tage bei 30 °C und unter Bewegen (200 Upm) in einem befeuchteten Schüttelschrank inkubiert. Nach dem Abbruch der Fermentation wurde der Gehalt an Lysin per HPLC bestimmt. Die HPLC Analysen wurden mit Geräten der 1100 Series LC von Agilent durchgeführt. Die Bestimmung der Aminosäurekonzentration erfolgte mittels Hochdruckflüssigkeitschromatographie auf einer Agilent 1100 Series LC System HPLC. Eine Vorsäulenderivatisierung mit Ortho-Pthalaldehyd erlaubt die Quantifizierung der gebildeten Aminosäuren, die Auftrennung des Aminosäuregemisches findet auf einer Hypersil AA-Säule (Agilent) statt.

Die Ergebnisse sind in Tabelle 5 zusammengestellt.

**Tabelle 5: Lysinproduktion (Mittelwerte)**

| Fermentationszeit | Lysin [g/l] |
|---|---|
| 45 h | 11,5 |
| 70 h | 12,8 |
| Kontrolle (45 h) | 11,1 |

### Beispiel 2

Verflüssigtes Maismehlhydrolysat wurde in Schüttelkolbenversuchen unter Verwendung von *Aspergillus niger* eingesetzt.

### I) Verflüssigung

Die Verflüssigung wurde wie bei Beispiel 1 unter I) beschrieben durchgeführt.

### II) Fermentation mit Aspergillus niger

### Stamm

Ein *Aspergillus niger* Phytase-Produktionsstamm mit 6 Kopien des phyA-Gens aus *Aspergillus ficuum* unter der Kontrolle des glaA-Promotors wurde analog zur im Detail in WO98/46772 beschriebenen Herstellung von NP505-7 erzeugt. Als Kontrolle wurde ein Stamm mit 3 modifizierten glaA Ampikons (analog ISO505), jedoch ohne integrierte phyA-Expressionskassetten verwendet.

### Herstellunq des Inokulums

20 ml des Vorkulturmediums (siehe Tabelle 6) in 100 ml Erlenmeyerkolben mit einer Schikane werden jeweils mit 100 µl einer Gefrierkultur angeimpft und 24 h bei 34 °C unter Bewegen (170 Upm) in einem befeuchteten Schüttelschrank inkubiert.

**Tabelle 6: Zusammensetzung des Vorkulturmediums**

| Inhaltsstoff | Konzentration |
|---|---|
| Glucose | 30,0 g/l |
| Pepton aus Casein | 10,0 g/l |
| Hefeextrakt | 5,0 g/l |
| KH₂PO₄ | 1,0 g/l |
| MgSO₄ x 7 H₂O | 0,5 g/l |
| ZnCl₂ | 30 mg/l |
| CaCl₂ | 20 mg/l |
| MnSO₄ x 1 H₂O | 9 mg/l |
| FeSO₄ x 7H₂O | 3 mg/l |
| Tween 80 | 3,0 g/l |
| Penicillin | 50000 IU/l |
| Streptomycin | 50 mg/l |
| pH* | 5,5 |

| | |
|---|---|
| * mit verdünnter Schwefelsäure eingestellt | |

50 ml des Hauptkulturmediums (siehe Tabelle 7) in 250 ml Erlenmeyerkolben mit einer Schikane werden jeweils mit 5ml Vorkultur angeimpft

### Herstellunq der Fermentationsbrühe

Die Zusammensetzungen des Kolbenmediums sind in Tabelle 7 aufgeführt. Aus jeder Probe wurden zwei Kolben angesetzt.

**Tabelle 7: Kolbenmedien**

| Maismehlhydrolysat | 200 g/l |
|---|---|
| Pepton aus Casein | 25,0 g/l |
| Hefeextrakt | 12,5 g/l |
| KH₂PO₄ | 1,0 g/l |
| K₂SO₄ | 2,0 g/l |
| MgSO₄ x 7 H₂O | 0,5 g/l |
| ZnCl₂ | 30 mg/l |
| CaCl₂ | 20 mg/l |
| MnSO₄ x 1 H₂O | 9 mg/l |
| FeSO₄ x 7 H₂O | 3 mg/l |
| Penicillin | 50000 IU/I |
| Streptomycin | 50 mg/l |
| pH* | 5,6 |

| | |
|---|---|
| * mit verdünnter Schwefelsäure einzustellen | |

Nach dem Animpfen wurden die Kolben 6 Tage bei 34 °C und unter Bewegen (170 Upm) in einem befeuchteten Schüttelschrank inkubiert. Nach dem Abbruch der Fermentation wurde die Phytaseaktivität mit Phytinsäure als Substrat und auf einem geeignetem Phytaseaktivitätsniveau (Standard: 0,6 U/ml) in 250 mM Essigsäure/Natriumacetat/Tween 20 (0,1 Gew.-%), pH 5,5 Puffer bestimmt. Der Assay wurde standardisiert für die Anwendung in Mikrotiterplatten (MTP). 10 µl der Enzymlösung wurden mit 140 µl 6,49 mM Phytatlösung in 250 mM Natriumacetatpuffer, pH 5,5 (Phytat: Dodecanatriumsalz der Phytinsäure) gemischt. Nach einer Stunde Inkubation bei 37 °C wurde die Reaktion durch Zugabe des gleichen Volumens (150 µl) Trichloressigsäure gestoppt. Ein Aliquot dieser Mischung (20 µl) wurde überführt in 280 µl einer Lösung, die 0,32 N H₂SO4, 0,27 Gew.-% Ammoniummolybdat und 1,08 Gew.-% Ascorbinsäure enthält. Anschließend erfolgte eine 25-minütige Inkubation bei 50 °C. Die Absorption der blau gefärbten Lösung wurde bei 820 nm gemessen. Die Ergebnisse sind in Tabelle 8 zusammengestellt.

**Tabelle 8: Phytaseaktivität nach Abbruch der Fermentation**

| **Kolben** | *Phytaseaktivität [FTU*/*ml]* |
|---|---|
| 1 | 569 |
| 2 | 696 |
| Kontrolle | 393 |

## Patentansprüche

1. Verfahren zur Herstellung wenigstens einer organischen Verbindung mit mindestens 3 C-Atomen oder mit mindestens 2 C-Atomen und mindestens 1 N-Atom durch Fermentation, umfassend die folgenden Schritte:
a1) Vermahlen einer Stärkequelle, wobei man ein Mahlgut erhält, das wenigstens einen Teil der nicht-stärkehaltigen festen Bestandteile der Stärkequelle enthält;
a2) Suspendieren des Mahlguts mit einer wässrigen Flüssigkeit und Verflüssigen des in der wässrigen Flüssigkeit enthaltenen Mahlguts in Gegenwart mindestens eines Stärke verflüssigenden Enzyms, wobei man ein wässriges Dextrin-haltiges Medium (1) erhält, das wenigstens einen Teil der nicht-stärkehaltigen festen Bestandteile der Stärkequelle umfasst; und
b) Verwendung des wässrigen Dextrin-haltigen Mediums (1) in einer Fermentation zur Kultivierung eines Mikroorganismus, welcher zur Überproduktion der organischen Verbindung befähigt ist und der ausgewählt ist unter Mikroorganismen, die Enzyme produzieren, welche Dextrine zu Monosacchariden hydrolysieren;
wobei Enzyme, die Dextrine zu Monosacchariden hydrolysieren, nicht oder in einer Menge von weniger als 0,001 Gew.%, bezogen auf das Gesamtgewicht der eingesetzten Stärkequelle, zugegeben werden.

2. Verfahren nach Anspruch 1, wobei man die Suspension des Mahlguts in der wässrigen Flüssigkeit auf eine Temperatur oberhalb der Verkleisterungstemperatur der in der Stärkequelle enthaltenen Stärke erhitzt.

3. Verfahren nach Anspruch 2, wobei das Erhitzen in Gegenwart des Stärkeverflüssigenden Enzyms erfolgt.

4. Verfahren nach einem der vorherigen Ansprüche, wobei man mindestens eine Teilmenge des Mahlguts im Verlauf der Verflüssigung kontinuierlich oder diskontinuierlich zu der wässrigen Flüssigkeit gibt.

5. Verfahren nach einem der vorherigen Ansprüche, wobei man das Mahlgut in einer solchen Menge in der wässrigen Flüssigkeit suspendiert und in dieser verflüssigt, dass das erhaltene wässrige Dextrin-haltige Medium (1) einen Trockenmassegehalt von mindestens 50 Gew.-%, bezogen auf das Gesamtgewicht des Mediums (1), aufweist.

6. Verfahren nach einem der vorherigen Ansprüche, wobei man das Mahlgut in einer solchen Menge in der wässrigen Flüssigkeit suspendiert und in dieser verflüssigt, dass das erhaltene wässrige Dextrin-haltige Medium (1) eine Konzentration an Glukoseäquivalenten von mindestens 40 Gew.-%, bezogen auf das Gesamtgewicht des Mediums (1), aufweist.

7. Verfahren nach einem der vorherigen Ansprüche, umfassend zusätzlich die folgenden Schritte:
b1) Kultivieren des zur Überproduktion der organischen Verbindung befähigten Mikroorganismus in einem wässrigen Fermentationsmedium (2); und
b2) Zugabe des Dextrin-haltigen Mediums (1) zu dem Fermentationsmedium (2), in welchem die im Medium (1) enthaltenen Dextrine durch die die organische Verbindung überproduzierenden Mikroorganismen verstoffwechselt werden.

8. Verfahren nach Anspruch 7, wobei das Fermentationsmedium (2) in Schritt b1) im Wesentlichen das Medium (1), die zur Überproduktion der organischen Verbindung befähigten Mikroorganismen, übliche Medienbestandteile und gegebenenfalls Wasser zur Verdünnung umfasst.

9. Verfahren nach Anspruch 7 oder 8, wobei man in Schritt b1) eine solche Menge des Mediums (1) zum Ansetzen des Fermentationsmediums (2) verwendet, dass die Gesamtkonzentration an Zucker im Fermentationsmedium (2) im Bereich von 6 bis 30 Gew.-%, gerechnet als Glukoseäquivalente und bezogen auf das Gesamtgewicht des Fermentationsmediums (2), liegt.

10. Verfahren nach einem der vorherigen Ansprüche, wobei man in Schritt a1) als Stärkequelle Getreidekörner verwendet.

11. Verfahren nach einem der vorherigen Ansprüche, wobei das Mahlgut mindestens 20 Gew.-% der gesamten nicht-stärkehaltigen festen Bestandteile der Stärkequelle umfasst.

12. Verfahren nach einem der vorherigen Ansprüche, wobei die hergestellte organische Verbindung unter gegebenenfalls Hydroxylgruppen tragenden Mono-, Di- und Tricarbonsäuren mit 3 bis 10 C-Atomen, proteinogenen und nicht-proteinogenen Aminosäuren, Purinbasen, Pyrimidinbasen; Nukleosiden, Nukleotiden, Lipiden; gesättigten und ungesättigten Fettsäuren; Diolen mit 4 bis 10 C-Atomen, mehrwertigen Alkoholen mit 3 oder mehr Hydroxylgruppen, langkettigen Alkoholen mit wenigstens 4 C-Atomen, Kohlenhydraten, aromatischen Verbindungen, Vitaminen, Provitaminen, Cofaktoren, Nutrazeutika, Proteinen, Carotenoiden, Ketonen mit 3 bis 10 C-Atomen, Lactonen, Biopolymeren und Cyclodextrinen ausgewählt ist.

13. Verfahren nach einem der vorherigen Ansprüche, wobei man wenigstens ein mikrobielles Stoffwechselprodukt aus der Fermentationsbrühe abreichert oder isoliert und anschließend die flüchtigen Bestandteile der Fermentationsbrühe weitgehend entfernt, wobei man eine feste oder halbfeste Proteinzusammensetzung erhält.

14. Verfahren nach einem der Ansprüche 1 bis 12, wobei man die flüchtigen Bestandteile der Fermentationsbrühe ohne vorherige Isolierung oder Abreicherung eines nichtflüchtigen mikrobiellen Stoffwechselprodukts, und gegebenenfalls ohne vorherige Abtrennung fester Bestandteile, mindestens teilweise entfernt, wobei man eine feste Formulierung eines nichtflüchtigen mikrobiellen Stoffwechselprodukts erhält.

## Claims

1. A process for the production of at least one organic compound having at least 3 C atoms or having at least 2 C atoms and at least one N atom by means of fermentation, comprising the following steps:
a1) milling a starch feedstock, thus obtaining a millbase which comprises at least part of the nonstarchy solid constituents of the starch feedstock;
a2) suspending the millbase in an aqueous liquid and liquefying the millbase present in the aqueous liquid in the presence of at least one starch-liquefying enzyme, obtaining an aqueous dextr_in-containing medium (1) which comprises at least a part of the nonstarchy solid constituents of the starch feedstock; and
b) using the aqueous dextrin-containing medium (1) in a fermentation for culturing a microorganism which is capable of overproducing the organic compound and which is selected among microorganisms which produce enzymes which hydrolyze dextrins to monosaccharides;
enzymes which hydrolyze dextrins to monosaccharides being added in an amount of less than 0.001% by weight based on the total weight of the starch feedstock employed, or not at all.

2. The process according to claim 1, wherein the suspension of the millbase in the aqueous liquid is heated to a temperature above the gelatinization temperature of the starch present in the starch feedstock.

3. The process according to claim 2, wherein heating is carried out in the presence of the starch-liquefying enzyme.

4. The process according to any of the preceding claims, wherein at least one portion of the millbase is added continuously or batchwise to the aqueous liquid during the liquefaction step.

5. The process according to any of the preceding claims, wherein the millbase is suspended in such an amount in the aqueous liquid and liquefied therein that the resulting aqueous dextrin-containing medium (1) has a dry-matter content of at least 50% by weight based on the total weight of the medium (1).

6. The process according to any of the preceding claims, wherein the millbase is suspended in such an amount in the aqueous liquid and liquefied therein that the resulting aqueous dextrin-containing medium (1) has a glucose equivalent concentration of at least 40% by weight based on the total weight of the medium (1).

7. The process according to any of the preceding claims, additionally comprising the following steps:
b1) culturing, in an aqueous fermentation medium (2), the microorganism which is capable of overproducing the organic compound; and
b2) addition of the dextrin-containing medium (1) to the fermentation medium (2) in which the dextrins present in the medium (1) are metabolized by the microorganisms which overproduce the organic compound.

8. The process according to claim 7, wherein the fermentation medium (2) in step b1) comprises essentially the medium (1), the microorganisms which are capable of overproducing the organic compound, conventional media constituents and, optionally, water for dilution.

9. The process according to claim 7 or 8, wherein, in step b1), such an amount of the medium (1) is used for making up the fermentation medium (2) that the total sugar concentration in the fermentation medium (2) is in the range from 6 to 30% by weight, calculated as glucose equivalents and based on the total weight of the fermentation medium (2).

10. The process according to any of the preceding claims, wherein cereal kernels are used as starch feedstock in step a1).

11. The process according to any of the preceding claims, wherein the millbase comprises at least 20% by weight of all of the nonstarchy solid constituents of the starch feedstock.

12. The process according to any of the preceding claims, wherein the organic compound which has been produced is selected among mono-, di- and tricarboxylic acids which optionally have hydroxyl groups attached to them and which have 3 to 10 carbon atoms, among proteinogenic and nonproteinogenic amino acids, purine bases, pyrimidine bases; nucleosides, nucleotides, lipids; saturated and unsaturated fatty acids; diols having 4 to 10 carbon atoms, polyhydric alcohols having 3 or more hydroxyl groups, long-chain alcohols having at least 4 carbon atoms, carbohydrates, aromatic compounds, vitamins, provitamins, cofactors, nutraceuticals, proteins, carotenoids, ketones having 3 to 10 carbon atoms, lactones, biopolymers and cyclodextrins.

13. The process according to any of the preceding claims, wherein at least one microbial metabolite is depleted or isolated from the fermentation liquor and the volatile constituents of the fermentation liquor are subsequently substantially removed, a solid or semisolid protein composition being obtained.

14. The process according to any of claims 1 to 12, wherein at least some of the volatile constituents of the fermentation liquor are removed without previous isolation or depletion of a nonvolatile microbial metabolite and, optionally, without previous removal of solid constituents, a solid formulation of a nonvolatile microbial metabolite being obtained.

## Revendications

1. Procédé de production d'au moins un composé organique comprenant au moins 3 atomes de carbone ou au moins 2 atomes de carbone et 1 atome d'azote par fermentation, comprenant les étapes suivantes:
a1) moudre une source d'amidon, dans lequel on obtient un produit moulu qui contient au moins une partie des composants solides sans amidon de la source d'amidon;
a2) mettre en suspension le produit moulu avec un liquide aqueux et liquéfier le produit moulu contenu dans le liquide aqueux en présence d'au moins une enzyme liquéfiant l'amidon, dans lequel on obtient un milieu (1) contenant de la dextrine, qui comprend au moins une partie des composants solides sans amidon de la source d'amidon; et
a3) utiliser le milieu aqueux contenant de la dextrine (1) dans une fermentation pour la culture d'un microorganisme, qui est capable de produire en excès le composé organique et qui est choisi parmi des microorganismes qui produisent des enzymes, qui hydrolysent les dextrines en monosaccharides;
dans lequel des enzymes, qui hydrolysent les dextrines en monosaccharides, ne sont pas ajoutées ou le sont en une quantité de moins de 0,001 % en poids, rapportée au poids total de la source d'amidon utilisée.

2. Procédé selon la revendication 1, dans lequel on chauffe la suspension du produit moulu dans le liquide aqueux à une température supérieure à la température de gélatinisation de l'amidon contenu dans la source d'amidon.

3. Procédé selon la revendications 2, dans lequel on effectue le chauffage en présence de l'enzyme liquéfiant l'amidon.

4. Procédé selon l'une quelconque des revendications précédentes, dans lequel on ajoute au moins une quantité partielle du produit moulu au liquide aqueux de façon continue ou discontinue au cours de la liquéfaction.

5. Procédé selon l'une quelconque des revendications précédentes, dans lequel on met le produit moulu en suspension dans le liquide aqueux et on le liquéfie dans celui-ci en une quantité telle que le milieu aqueux contenant de la dextrine obtenu (1) présente une teneur en masse sèche d'au moins 50 % en poids, rapportée au poids total du milieu (1).

6. Procédé selon l'une quelconque des revendications précédentes, dans lequel on met le produit moulu en suspension dans le liquide aqueux et on le liquéfie dans celui-ci en une quantité telle que le milieu aqueux contenant de la dextrine obtenu (1) présente une concentration en glucose équivalent d'au moins 40 % en poids, rapportée au poids total du milieu (1).

7. Procédé selon l'une quelconque des revendications précédentes, comprenant en outre les étapes suivantes:
b1) cultiver le microorganisme capable de produire en excès le composé organique, dans un milieu de fermentation aqueux (2); et
b2) ajouter le milieu contenant de la dextrine (1) au milieu de fermentation (2), dans lequel les dextrines contenues dans le milieu (1) sont dégradées par les microorganismes qui produisent en excès le composé organique.

8. Procédé selon la revendication 7, dans lequel le milieu de fermentation (2) dans l'étape b1) comprend essentiellement le milieu (1), les microorganismes capables de produire en excès le composé organique, des composants usuels du milieu et éventuellement de l'eau pour la dilation.

9. Procédé selon la revendication 7 ou 8, dans lequel on utilise à l'étape b1) une telle quantité du milieu (1) pour préparer le milieu de fermentation (2), que la concentration totale en sucre dans le milieu de fermentation (2) se situe dans la plage de 6 à 30 % en poids, calculée en glucose équivalent et rapportée au poids total du milieu de fermentation (2).

10. P.rocédé selon l'une quelconque des revendications précédentes, dans lequel on utilise à l'étape a1) des grains de céréales comme source d'amidon.

11. Procédé selon l'une quelconque des revendications précédentes, dans lequel le produit moulu comprend au moins 20 % en poids de tous les composants solides sans amidon de la source d'amidon.

12. Procédé selon l'une quelconque des revendications précédentes, dans lequel on sélectionne le composé organique produit parmi des acides mono-, di- ou tricarboxyliques avec 3 à 10 atomes de carbone portant éventuellement des groupes hydroxyle, des acides aminés protéinogènes et non protéinogènes, des bases puriques, des bases pyrimidiques; des nucléosides, des nucléotides, des lipides; des acides gras saturés et insaturés; des diols avec 4 à 10 atomes de carbone, des alcools polyvalents avec 3 groupes hydroxyle ou plus, des alcools à longue chaîne avec au moins 4 atomes de carbone, des hydrates de carbone, des composés aromatiques, des vitamines, des provitamines, des cofacteurs, des nutraceutiques, des protéines, des caroténoïdes, des cétones avec 3 à 10 atomes de carbone, des lactones, des biopolymères et des cyclodextrines.

13. Procédé selon l'une quelconque des revendications précédentes, dans lequel on appauvrit ou on isole au moins un produit de dégradation microbien hors du bouillon de fermentation et on élimine ensuite largement les composants volatils du bouillon de fermentation, dans lequel on obtient une composition de protéines solide ou semi-solide.

14. Procédé selon l'une quelconque des revendications 1 à 12, dans lequel on élimine au moins partiellement les composants volatils du bouillon de fermentation sans isolement ou appauvrissement préalable d'un produit de dégradation microbien non volatil, et éventuellement sans séparation préalable de composants solides, dans lequel on obtient une formulation solide d'un produit de dégradation microbien non volatil.
